# EUROPÄISCHE PATENTSCHRIFT

(11) **EP 1 402 882 B1**
(45) Veröffentlichungstag und Bekanntmachung des Hinweises auf die Patenterteilung: **03.03.2010**
(21) Anmeldenummer: 03021187.4
(22) Anmeldetag: 24.09.2003
(51) Int. Cl.: A61K 6/08, A61K 8/24

(54) **Säurehaltige Desensibilisierungsmittel für Zähne**
Acid-containing tooth desensitising material
Désensibilisant pour les dents contenant un acide

(30) Priorität: 27.09.2002 DE 10245212
(43) Veröffentlichungstag der Anmeldung: 31.03.2004
(73) Patentinhaber: Ivoclar Vivadent AG, 9494 Schaan (LI)
(72) Erfinder: Huwig, Alexander Karl, Dr., 68729 Schwetzingen (DE); Moszner, Norbert, Prof. Dr., 9492 Triesen (LI); Zeuner, Frank, Dr., 9488 Schellenberg (LI); Rheinberger, Volker, Dr., 9490 Vaduz (LI); Bolis, Carlo, CH-7000 Chur (CH)
(74) Vertreter: UEXKÜLL & STOLBERG

(56) Entgegenhaltungen:
- WO-A-87/07891
- WO-A-98/44964
- WO-A-99/52498
- GB-A- 1 394 126
- US-A- 4 224 307
- US-A- 5 190 946
- US-A- 5 505 933

## Beschreibung

Die Erfindung betrifft säurehaltige Zusammensetzungen, die sich insbesondere zur Desensibilisierung von Zähnen eignen.

Eine Überempfindlichkeit oder Hyperästhesie der Zähne gegenüber physikalischen (Kälte, Kontakt, Wärme), chemischen (Säure) oder osmotischen Reizen ist meist auf eine Freilegung des Dentins, insbesondere im Bereich der Zahnhälse und der Wurzelbereiche zurückzuführen. Ursache hierfür kann das Verschwinden des schützenden Zahnschmelzes als Folge von Erosion, Abrasion oder Abbrechen oder durch das Entblößen der Wurzeloberfläche durch Zahnfleischschwund oder in Folge einer Parodontalbehandlung sein.

Es wird angenommen, daß der Schmerz durch Bewegung der in den Tubili des Dentins vorhandenen Flüssigkeit (Dentinliquor) ausgelöst wird. Hierdurch kommt es zu einer kurzzeitigen Stauchung oder Dehnung der in der Pulpa vorhandenen Nervenzellen, die hierauf durch das Auslösen und Weiterleiten eines Schmerzsignals reagieren.

Die Entstehung eines Schmerzsignals in einer Nervenzelle ist an einen Kaliumionengradienten gebunden. Die Kaliumionenkonzentration ist im Ruhezustand innerhalb der Nervenzelle größer als außerhalb. Bei einem Reiz werden Ionenkanäle geöffnet, die die Kaliumionen nach außen strömen lassen und so das Schmerzsignal auslösen. Durch das Einstellen einer hohen Kaliumionenkonzentration auf der Außenseite der Nervenzelle kann das Ausströmen von Kaliumionen aus der Zelle und damit die Entstehung und Weiterleitung des Schmerzsignals unterbunden werden. Bei der Therapie überempfindlicher Zähne werden jedoch durch das Erhöhen der extrazellulären Kaliumionenkonzentration, beispielsweise durch kaliumionenhaltige Mundspüllösungen oder Zahnpasten, nur kurzfristige Erfolge erzielt.

Neben der Erhöhung der extrazellulären Kaliumionenkonzentration läßt sich eine Desensibilisierung empfindlicher Zähne durch den Verschluß der Dentintubuli erreichen. Hierdurch werden Flüssigkeitsbewegungen in den Tubili und damit Reizungen der Nerverzellen verhindert.

Die Firma Septodont Pharm-Dental Handelsgesellschaft m.b.H. vertreibt unter der Bezeichnung Isodan® ein Produkt, das neben Kaliumnitrat und Natriumfluorid Hydroxyethylmethacrylat (HEMA) enthält und das durch Koagulation der Tubuliproteine Flüssigkeitsbewegungen innerhalb der Tubuli verhindern soll.

In dem Produkt Gluma® der Firma Heraeus Kulzer werden die proteinfällenden Eigenschaften von HEMA mit den vernetzenden Eigenschaften von Glutaraldehyd kombiniert. Glutaraldehyd soll die gefällten Tubuliproteine kovalent mit dem im Dentin enthaltenen Kollagen verbinden.

Die Anwendung von Glutaraldehyd in medizinischen Produkten ist wegen seiner alkylierenden Wirkung unter gesundheitlichen Gesichtspunkten nicht unbedenklich. Zudem wird mit den bekannten Materialien selbst bei der Verwendung von Vernetzungsmitteln nur eine unzureichende Desensibilisierung der Zähne erzielt.

Die WO 99/52498 offenbart ein Verfahren zur Verringerung der Sensibilität von Dentin beim dem man die Dentinoberfläche zunächst mit einer Säure behandelt und dann eine Zusammensetzung aufträgt, die ein saures Oxalat und gegebenenfalls ein Verdikkungsmittel wie Hydroxyethylcellulose enthält. Anschließend kann gegebenenfalls ein Bindemittel zur Versiegelung der Dentinoberfläche aufgetragen werden.

Die US 5,505,933 offenbart orale Zusammensetzungen wie z.B. Zahnpasten, die ein Polyphosphat als Antizahnsteinmittel und ein Kaliumsalz zur Desensibilisierung der Zähne enthalten. Die Zusammensetzungen enthalten vorzugsweise auch ein anionisches, polymeres Polycarboxylat, das zur Stabilisierung des Polyphosphats dient. Außerdem können sie Phosphonoverbindungen als weitere Antizahnsteinmittel enthalten. Die Zusammensetzungen haben einen pH-Wert im Bereich von 6 bis 8.

Die Aufgabe der Erfindung besteht in der Bereitstellung von Desensibilisierungsmitteln für Zähne, die eine langanhaltende Desensibilisierung ermöglichen.

Diese Aufgabe wird durch Zusammensetzungen gelöst, die neben einer Säure ein organisches Polymer enthalten, das Carboxylund/oder Hydroxylgruppen aufweist.

Carboxylgruppenhaltige Polymere stellen keine Säuren im Sinne der Erfindung dar. Unter Säuren werden erfindungsgemäß nicht polymere Verbindungen verstanden. Als Säuren eignen sich besonders organische Säuren, wie Carbonsäuren, Sulfonsäuren und insbesondere Phosphonsäuren.

Erfindungsgemäß sind solche Säuren bevorzugt, die eine hohe Löslichkeit in Wasser oder in Wasser/Ethanol-Mischungen aufweisen. Unter einer hohen Löslichkeit wird eine Löslichkeit von 0,5 bis 50 Gew.-%, vorzugsweise 20 bis 50 Gew.-% in Wasser oder einer Mischung aus 50 Gew.-% Wasser und 50 Gew.-% Ethanol verstanden.

Die Säuren weisen neben proteinfällenden auch calciumfällende Eigenschaften auf. Protein- und calciumfällende Eigenschaften werden in standardisierten Tests ermittelt. Die Proteinfällung wird wie in Beispiel 6 beschrieben gemessen, wobei die zu untersuchende Säure zu der in Beispiel 5 beschriebenen Lösung (2. Komponente) in einer Konzentration von 0,2 M gegeben wird. Die säurehaltige Lösung wird mit der in Beispiel 6 beschriebenen Proteinlösung gemischt und die Masse des erhaltenen Präzipitats wird ermittelt. Erfindungsgemäß sind solche Säuren bevorzugt, die ein Pelletgewicht von > 25 mg, vorzugsweise > 30 mg und insbesondere > 40 mg ergeben. Die Obergrenze des Pelletgewichts wird durch die Massen der eingetzten Komponenten bestimmt. Säuren, die unter den beschriebenen Bedingungen eine quantitative Fällung ergeben, sind erfindungsgemäß besonders geeignet.

Zur Bestimmung der calciumfällenden Eigenschaften wird eine Mischung aus 5,0 g modifizierter Polyacrylsäure (PAA/GMA), 20 g Polyethylenglykol (PEG 1000 DMA), 5,0 g Hydroxypropylcellulose, 0,3 g Kaliumfluorid und 65,6 g Ethanol/Wasser (50:50) mit CaCl₂ auf eine Ca-Konzentration von 0,1 M eingestellt und 1 ml dieser Mischung anschließend mit 0,05 M der zu testenden Säure versetzt. Die säurehaltige Mischung wird kräfig geschüttelt, in einer Eppendorf-Zentrifuge bei 13.000 g für 5 Minuten zentrifugiert und bis zur Gewichtskonstanz getrocknet. Es sind solche Säuren bevorzugt, die in diesem Test ein Pelletgewicht von > 30 mg, vorzugsweise ≥ 40 mg, besonders bevorzugt 40 bis 60 mg und insbesondere 40 bis 50 mg ergeben. Als modifizierte Polyacrylsäure (PAA) wird Polyacrylsäure mit einem mittleren Molekulargewicht von etwa 30.000 g/mol verwendet, die durch Umsetzung mit 0,5 mol Glycidylmethacrylat (GMA) pro Acrylsäurebaustein im Polymer modifiziert wurde.

Bevorzugte Phosphonsäuren sind solche gemäß der folgenden Formel 1: 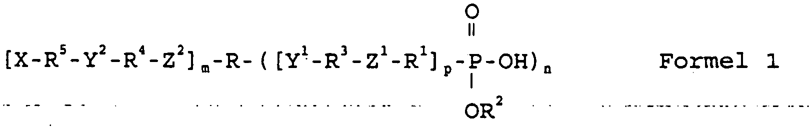 in der
- n: 1, 2, 3 oder 4 ist,
- m: 0, 1 oder 2 ist,
- p: 0 oder 1 ist,
- R: ein geradkettiger oder verzweigter aliphatischer Kohlenwasserstoffrest mit 1 bis 12 Kohlenstoffatomen oder ein aromatischer Kohlenwasserstoffrest mit 6 bis 12 Kohlenstoffatomen oder ein aliphatisch/arömatischer Kohlenwasserstoffrest mit 7 bis 16 Kohlenstoffatomen ist, der durch OH, NH₂ und/oder COOR⁶ substituiert sein kann,
- R¹: ein C₁- bis C₁₂-Alkylen-, C₄- bis C₁₂ Cycloalkylen-, C₆- bis C₁₂-Arylen- oder C₇- bis C₁₆-Alkylenarylenrest ist, der durch OH, NH₂ und/oder COOR⁶ substituiert sein kann, oder entfällt,
- R²: H, ein C₁- bis C₆-Alkyl- oder ein Phenylrest ist,
- R³, R⁴: unabhängig voneinander jeweils einen C₁- bis C₁₂-Alkylen- , C₆- bis C₁₂-Arylen- oder C₇- bis C₁₆-Alkylenarylenrest bedeuten, der durch Methyl, Phenyl oder Fluor substituiert sein kann, oder entfallen,
- R⁵: -CH=CR¹³- , ein Prop-1-en- 1,3-diyl-, C₁- bis C₆-Alkenylen-, C₃- bis C₉-Cycloalkylen-, C₁- bis C₆-Alkylen- oder Phenylenrest oder eine Gruppe mit der Formel 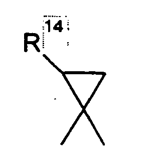 ist,
- R⁶: H, ein C₁- bis C₆-Alkyl- oder ein Phenylrest ist,
- Z¹,Z²: unabhängig voneinander jeweils CO-O, CO-NR⁷, O-CO-NH, O, NH, S bedeuten oder entfallen,
- Y¹,Y²: unabhängig voneinander jeweils O, CO-O, CO-NR⁸, O-CO-NH bedeuten oder entfallen,
- R⁷,R⁸: unabhängig voneinander jeweils H, oder einen C₁- bis C₆-Alkylrest bedeuten,
- X: H, CN, N(R⁹)₂, OR¹⁰, COOR¹¹ oder CONR₂¹² ist,
- R⁹, R¹⁰, R¹¹, R¹²: unabhängig voneinander jeweils H, einen C₁-bis C₁₀-Alkyl- oder einen Phenylrest bedeuten,
- R¹³: H oder ein Methylrest ist,
- R¹⁴: H oder ein C₁- bis C₁₀-Alkyl-, Vinyl- oder Phenylrest ist.

Unter Alkylenarylenresten werden Gruppen verstanden, die sowohl Alkylen- als auch Arylenreste enthalten, wie beispielsweie -CH₂-Phenylen-CH₂-.

Unabhängig voneinander wählbare bevorzugte Definition für die Variablen der Formel 1 sind:
- n: 1, 2 oder 3, insbesondere 1 oder 2 ist und/oder
- m: 1 ist und/oder
- p: 0 ist und/oder
- R: ein aliphatischer geradkettiger oder verzweigter ein- bis fünf-wertiger Alkanrest mit 1 bis 7 Kohlenstoffatomen, ein aromatischer Kohlenwasserstoffrest mit 6 Kohlenstoffatomen oder ein aliphatisch/aromatischer Kohlenwasserstoffrest mit 8 Kohlenstoffatomen ist und/oder
- R¹: ein Methylen- oder Ethylenrest ist oder entfällt und/oder
- R²: H, ein Methyl- oder Ethylrest ist und/oder
- R³, R⁴: unabhängig voneinander jeweils einen Methylen-, Ethylen-, Trimethylen-, p-Phenylen-, Ethyliden-, 1-Methylenethan-1,2-diylrest bedeuten oder entfallen und/oder
- R⁵: ein Methylen-, Ethylen-, Trimethylen-, Ethen-1,2-diyl-, Methylethylen-, Prop-1-en-1,3-diyl-, oder ein in 2-Stellung einfach substituierter Cyclopropylidenrest ist oder entfällt, insbesondere ein Methylen-, Ethylen- oder in 2-Stellung einfach substituierter Cyclopropylidenrest ist oder entfällt und/oder
- R⁶: H ist und/oder
- Z¹,Z²: unabhängig voneinander jeweils CO-O, O-CO-NH oder O bedeuten oder entfallen und/oder
- Y¹,Y²: unabhängig voneinander jeweils O, CO-O oder CO-NR⁸ bedeuten oder entfallen und/oder
- R⁷,R⁸: unabhängig voneinander jeweils H oder einen Methyl- oder Ethylrest bedeuten und/oder
- X: H, CN, COOR¹¹ oder CONR₂¹² ist und/oder
- R⁹, R¹⁰, R¹¹, R¹²: unabhängig voneinander jeweils H oder einen Methyl-, Ethyl- oder Phenylrest bedeuten und/oder
- R¹³: H oder ein Methylrest ist,
- R¹⁴: H oder ein Vinyl- oder Phenylrest ist.

Phosphonsäuren, bei denen mehrere und vorzugsweise alle Variablen eine der bevorzugten Definitionen aufweisen sind besonders bevorzugt.

Die Formel 1 schließt alle durch die genannten Substituenten möglichen Stereoisomere und ihre Mischungen, wie Racemate, ein.

Die Phosphonsäuren der Formel (1) lassen sich durch Synthese der entsprechenden Phosphonsäurediester und nachfolgende selektive Esterhydrolyse herstellen. Geeignete Alkylphosphonsäureester (APE) können auf unterschiedlichen Wegen erhalten werden. Eine bewährte Methode für die Herstellung von Alkanphosphonsäureestern ist die Michaelis-Arbusow-Reaktion (vgl. G. M. Kosolapoff, Org. Reactions 6 (1951) 273), bei der Trialkylphosphite, z.B. Triethylphosphit, und Halogenalkane miteinander umgesetzt werden, z.B.:

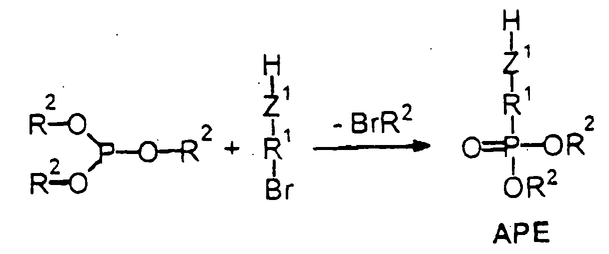

Konkretes Beispiel:

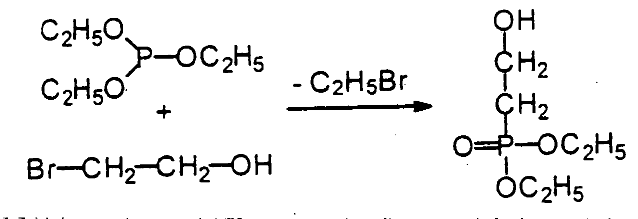

Dabei muß gegebenenfalls der Substituent Z geschützt werden.

Eine weitere Möglichkeit der Synthese von Hydroxyalkylphosphonsäureestern (Z = OH) besteht in der basenkatalysierten Anlagerung von Dialkylphosphiten an mono- oder difunktionelle Aldehyde oder Ketone (analog: F. Texier-Boullet, A. Foucaud, Synthesis, 1982, 916) :

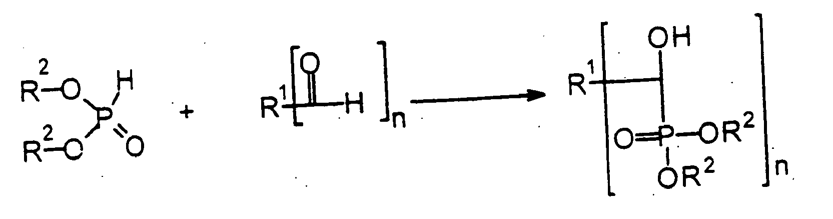

Konkretes Beispiel: 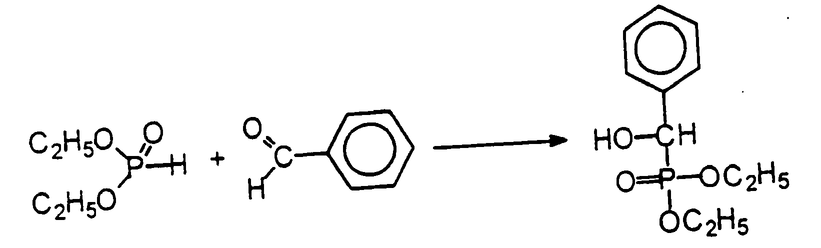 Acyloxyalkanphosphonsäurediester können aus Carbonsäurevinylestern durch Addition an Dialkylphosphite erhalten werden (DE-OS 2,127,821):

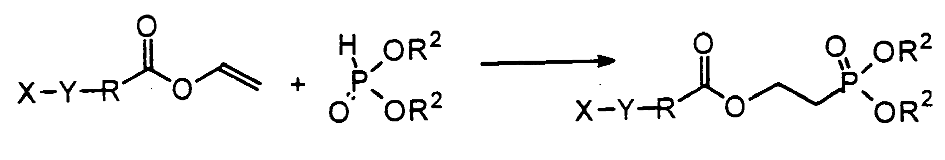

Konkretes Beispiel:

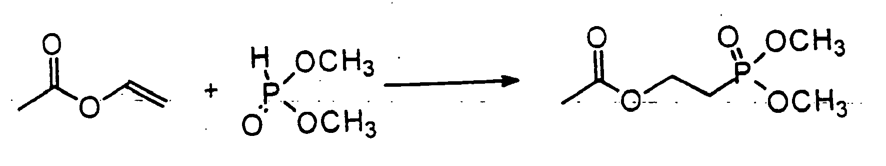

Die Phosphonsäuren der Formel (1) mit p = 0, n = 1, m = 1, R = -CH₂- (C=CH₂) -, R²= H, Y² = CO-O, CO-NR⁸ und X = H oder Y² entfällt und X = COOR⁸, Z², R⁴und R⁵ entfallen (ACPE) lassen sich durch Umsetzung von am Alkyl-Rest Z-funktionalisierten Alkylphosphonsäureestern APE (R² = Alkyl) mit Allylhalogeniden (U = Halogen, vor allem C1 oder Br) und nachfolgende Abspaltung der Alkylgruppen R² unter Anwendung der aus der organischen Chemie bekannten Methoden für die Knüpfung von C-C-, oder C-O- Bindungen (vgl. C. Weygand, G. Hilgetag, Organisch-chemische Experimentierkunst, Johann Ambrosius Bart Verlag, Leipzig 1970, S. 963f., 362f. und 657f.) herstellen.

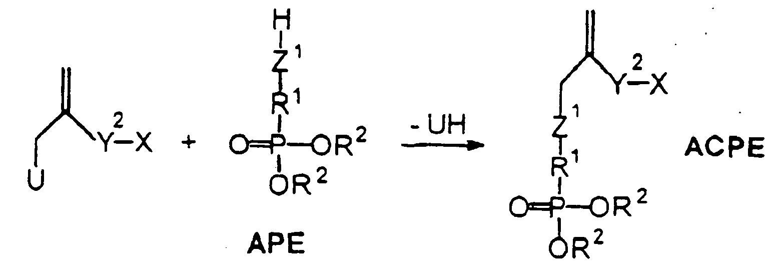

Konkretes Beispiel:

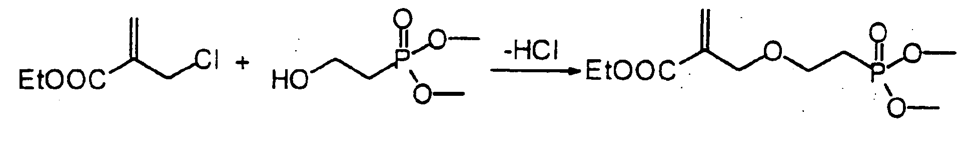

Die einfachen Allylhalogenide sind meist kommerziell erhältlich, aktivierte α-Halogenmethylacrylverbindungen können durch Reaktion von Acrylverbindungen mit Formaldehyd in Gegenwart von 1,4-Diazabicyclo[2.2.2]octan (DABCO) und nachfolgende Halogenierung mit anorganischen Säurechloriden, wie SOCl₂, PCl₃ oder PBr₃ erhalten werden (S.C.Warren, L.J.Mathias, J. Polymer Science, Part A: Polymer Chemistry 28 (1990) 1637), z.B.:

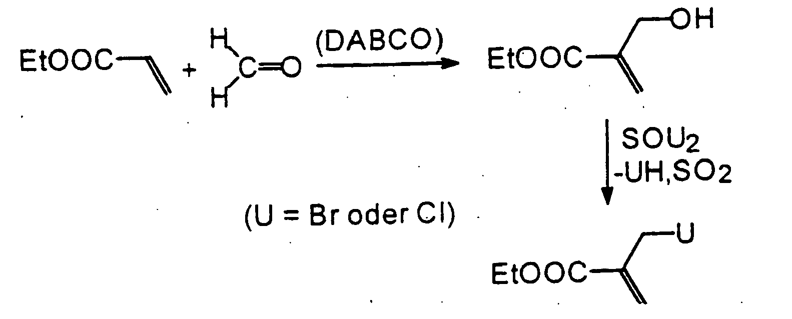

Phosphonsäuren der Formel (1) mit p = 0, n = 1, m = 1, R = -CH₂-(C=CH₂)-, R² = H, X = CO-W (W = N(R¹²)₂ oder OR¹¹) und Y², Z² , R⁴ und R⁵ entfallen (ACPA) lassen sich durch Umsetzung von Dialkoxyphosphorylacrylsäuren DPA mit Aminen oder Alkoholen in Gegenwart eines geeigneten Kondensationsmittels und nachfolgende Hydrolyse der Phosphonsäureestergruppen herstellen.

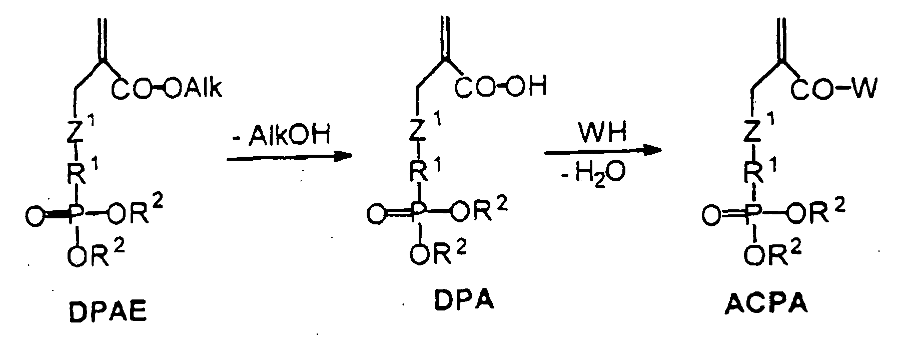

Als Kondensationsmittel können Carbodiimide bzw. Phosphoroxychlorid (Houben-Weyl, Bd. 15/2, Peptide; 4. Auflage, Georg Thieme Verlag, Stuttgart 1974, S. 103ff und 232ff) eingesetzt werden. Die verwendeten Dialkoxyphosphorylacrylsäuren DPA lassen sich aus den entsprechenden Dialkoxyphosphorylacrylsäurealkylestern DPAE (vgl. N. Moszner, F. Zeuner, U. K. Fischer, V. Rheinberger, Macromol. Chem. Phys. 200 (1999) 1062) durch selektive alkalische Hydrolyse herstellen.

Konkretes Beispiel:

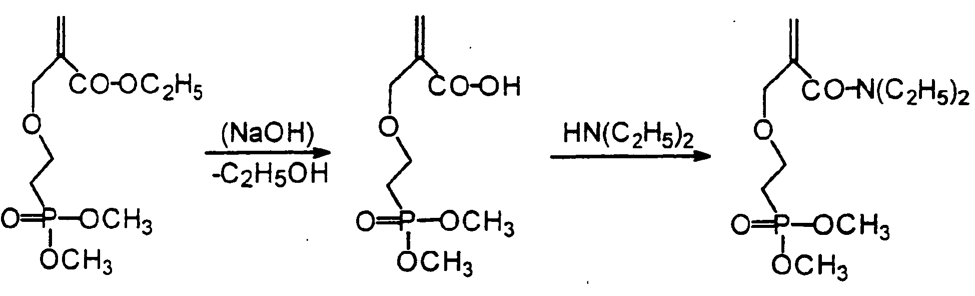

Darüber hinaus werden funktionalisierte Alkylphosphonsäureester durch Acylierung von am Rest Z¹ funktionalisierten Alkylphosphonsäureestern APE (R² = Alkyl) mit Carbonsäuren erhalten:

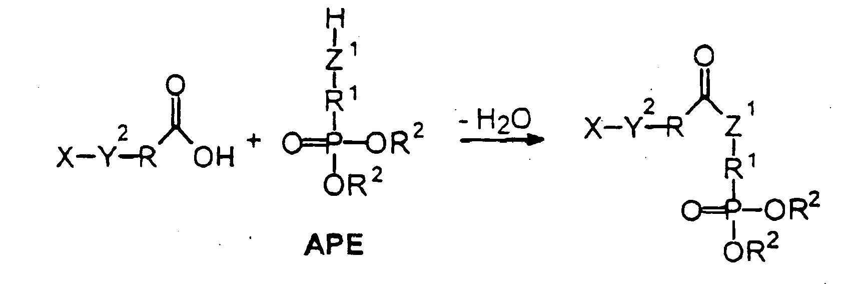

Konkretes Beispiel:

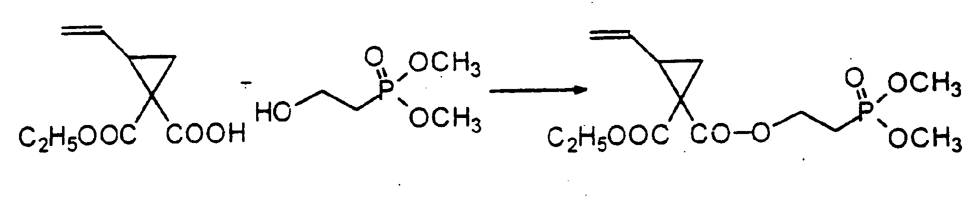

Entsprechende Vinylcyclopropan-1,1-dicarbonsäuremonoester können durch Umsetzung von Malonestern mit 1,4-Dibrom-but-2-enen und nach nachfolgende Hydrolyse des Diesters zum Monoester hergestellt werden (N. Moszner, F. Zeuner, V. Rheinberger, Macromol. Rapid Commun. 18 (1997) 775).

Die vollständige Hydrolyse der Phosphonsäurediester zu den entsprechenden Di-Phosphonsäuren erfolgt günstigerweise durch Silylierung mit Trialkylsilylhalogeniden, z.B. Trimethylsilylchlorid/(NaI oder NaBr), und nachfolgende Umsetzung mit Alkoholen oder Wasser (S. Freeman, J. Chem. Soc., Perkin Trans. 2, 1991, 263.). Zur selektiven Hydrolyse nur einer Esterfunktion werden sterisch gehinderte Silylhalogenide eingesetzt. Soll gleichzeitig eine Carbonsäureesterfunktion verseift werden, wird mit Alkalilauge hydrolysiert und nach erfolgter Hydrolyse die Phosphonsäure durch Ansäuern wieder freigesetzt. Die Salze erhält man durch Neutralisierung der Phosphonsäuren mit 1 Äquivalent des entsprechenden Hydroxids.

Konkretes Beispiel:

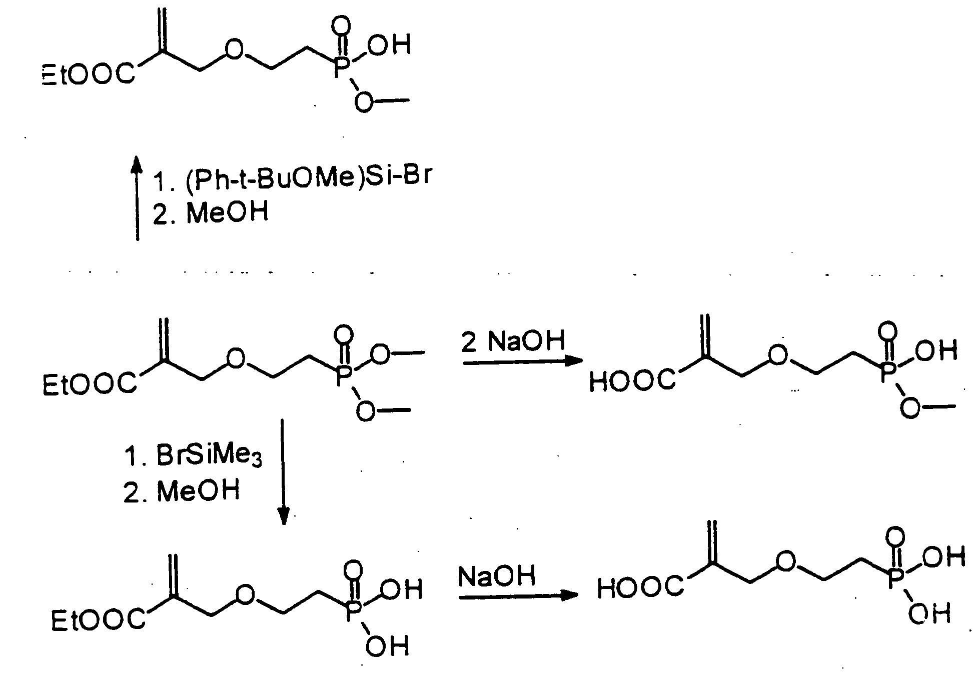

Beispiele für die erfindungsgemäßen Phosphonsäuren der Formel 1 sind u.a.: 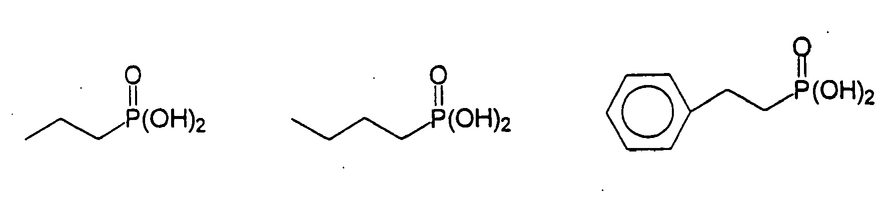 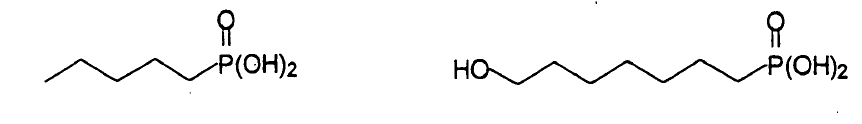 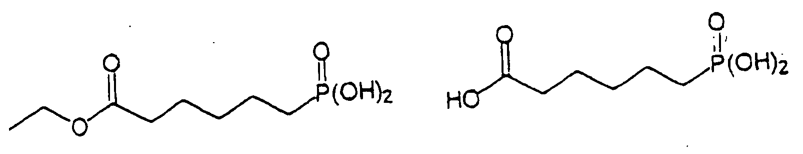 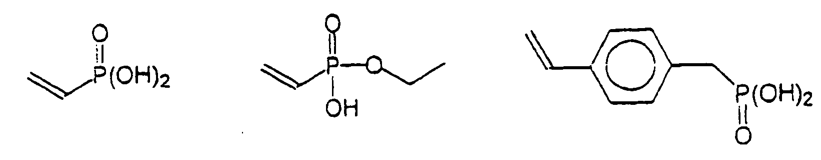 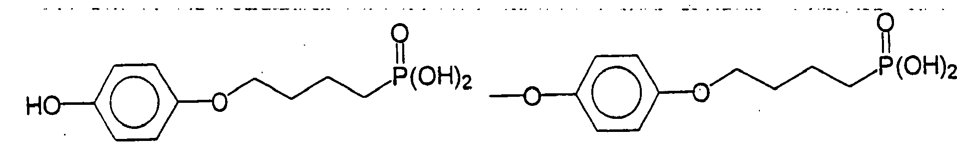 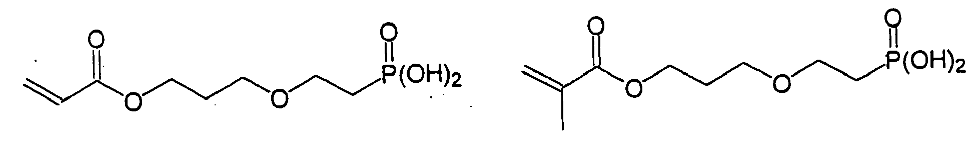 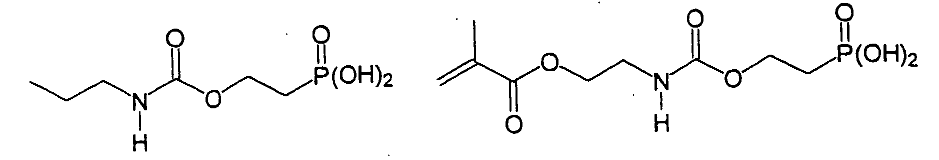 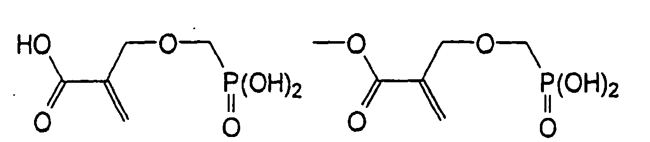 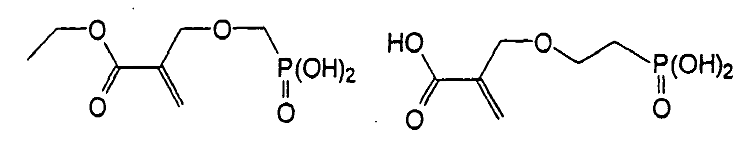 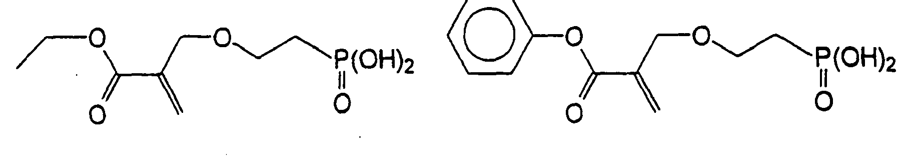 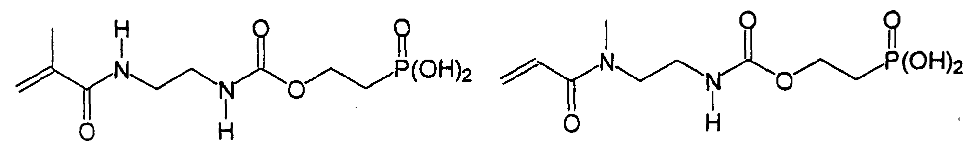 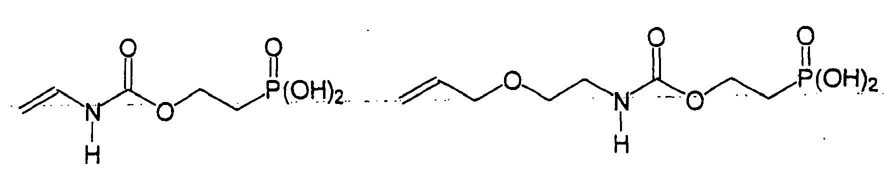 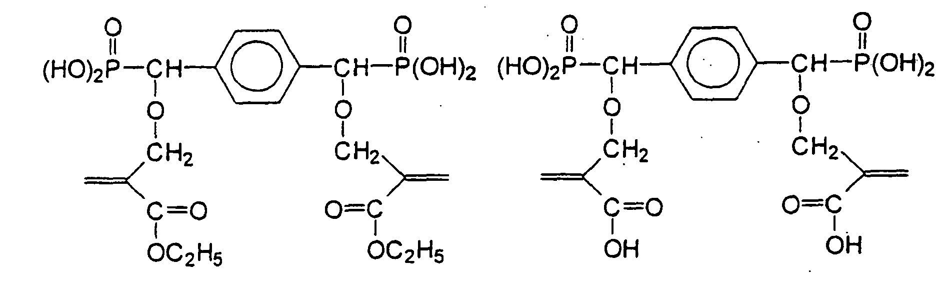 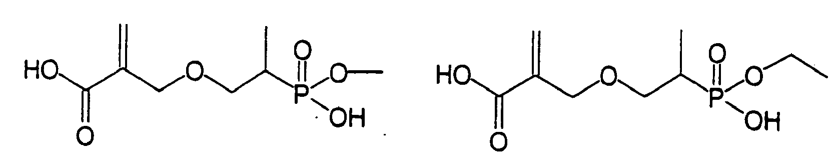 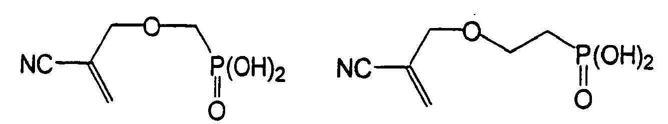 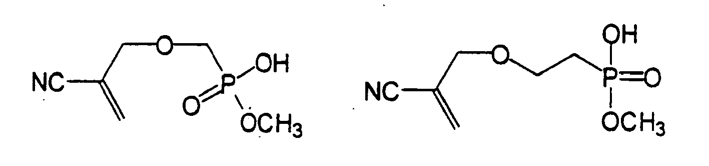 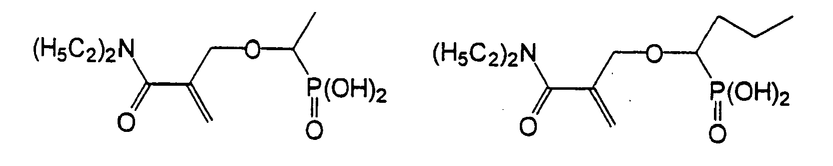 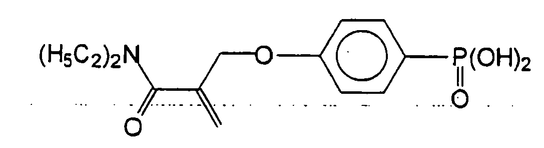 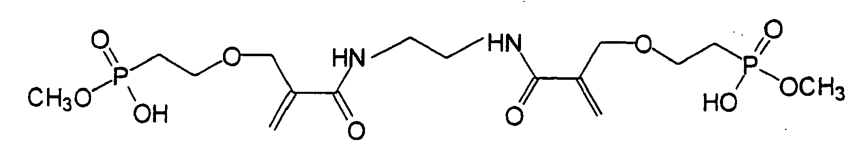 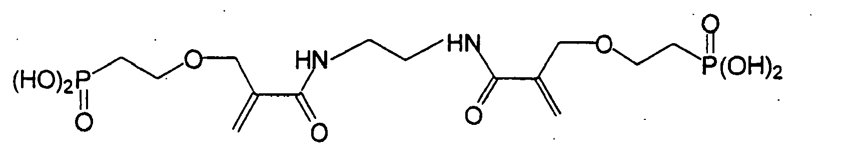 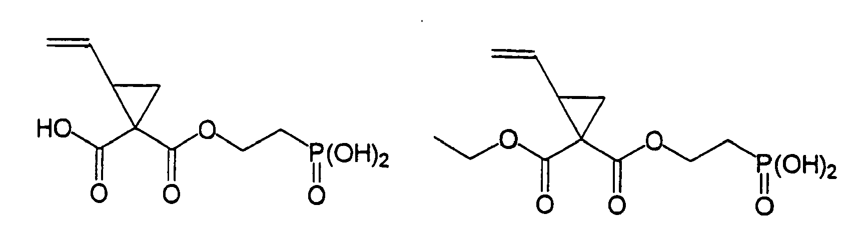 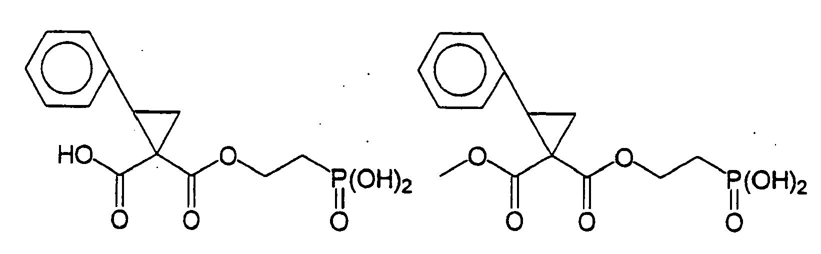 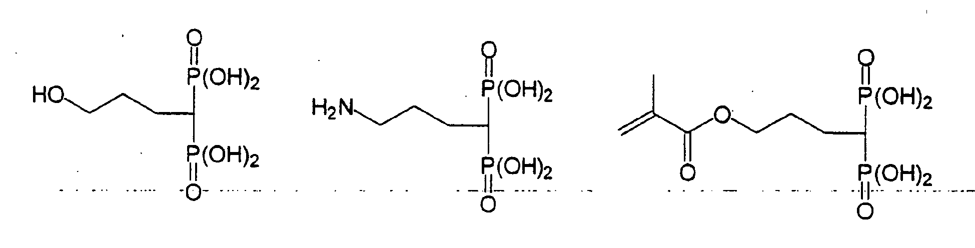 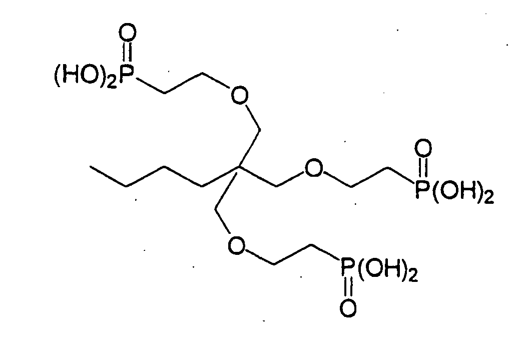 Geeignete Phosphonsäuren sind zum Teil kommerziell erhältlich, wie z.B. Vinylphosphonsäure (Clariant), 1-Hydroxyethan-diphosphonsäure, 2-Hydroxyethylphoshonsäure (Rhodia), oder lassen sich auf die oben beschriebene Weise herstellen.

Eine weitere Gruppe bevorzugter Phosphonsäuren bilden die in der DE 197 46 708 offenbarten Verbindungen. Hierbei handelt es sich um Säuren gemäß Formel 1 wobei
- n: 1 ist,
- m: 1 ist,
- p: 0 ist,
- R: ein C₁- bis C₃-Alkylen- oder Phenylenrest ist,
- R²: H ist,
- R⁴: ein verzweigter oder geradkettiger C₁- bis C₆-Alkylenrest ist, der durch 1 bis 2 Fluoratome und/oder 1 Phenylrest substituiert sein kann oder entfällt,
- R⁵: ein 1-Methylenethan-1,2-diylrest ist,
- Z²: entfällt,
- Y²: O ist oder entfällt,
- X: COOR¹¹ ist und
- R¹¹: H oder ein C₁- bis C₅-Alkyl- oder Phenylrest ist.

Weiter bevorzugt sind die in der DE 197 46 708 offenbarten Phosphonsäuren gemäß Formel 1 bei denen
- n: 2 ist,
- m: 2 ist,
- p: 1 ist,
- R: ein vierwertiger aliphatischer, aromatischer, oder aliphatisch-aromatischer Kohlenwasserstoffrest mit 2 bis 12 Kohlenstoffatomen ist,
- R¹: entfällt,
- R²: H ist,
- R³: ein C₁- bis C₃-Alkylen- oder Phenylenrest ist oder entfällt,
- R⁴: ein verzweigter oder geradkettiger C₁- bis C₆-Alkylenrest ist, der durch 1 bis 2 Fluoratome und/oder 1 Phenylrest substituiert sein kann oder entfällt,
- R⁵: ein 1-Methylenethan-1,2-diylrest ist,
- Z¹, Z²: entfallen,
- Y¹: entfällt,
- Y²: O ist oder entfällt,
- X: COOR¹¹ ist und
- R¹¹: H oder ein C₁- bis C₅-Alkyl- oder Phenylrest ist.

Die Herstellung dieser Phosphonsäuren wird in der DE 197 46 708 beschrieben.

Ganz besonders bevorzugte Phosphonsäuren sind 2-[4-(Dihydroxyphosphoryl)-2-oxa-butyl]-acrylsäure und 2-[4-(Methoxy-hydroxyphosphoryl)-2-oxa-butyl]-acrylsäure.

Ein Vorteil der erfindungsgemäß bevorzugten Phosphonsäuren ist darin zu sehen, daß sie eine selbstkonditionierende Wirkung haben, d.h. Dentinabrieb und Plaquereste vor dem Aufbringen der Desensibilisierungsmittel nicht entfernt werden müssen, das Dentin jedoch nicht anätzen.

Neben den Phosphonsäuren eignen sich Carbonsäuren, wie Maleinsäure und Trichloressigsäure, und besonders Sulfonsäuren, wie Sulfosalicylsäure (2-Hydroxy-5-sulfobenzoesäure), zur Herstellung der Zusammensetzungen.

Die genannten Säuren können allein oder als Mischung zur Herstellung der erfindungsgemäßen Zusammensetzungen eingesetzt werden. Bevorzugt sind Mischungen, die 1 bis 4, insbesondere 2 oder 3 verschiedene Säuren enthalten. Besonders bevorzugt sind Mischungen, die mindestens eine Phosphonsäure, ganz besonders bevorzugt zwei oder 3 Phosphonsäuren enthalten.

Als organische, carboxyl- und/oder hydröxylgruppenhaltige Polymere zur Kombination mit der Säure eignen sich besonders solche Polymere, die in Wasser oder Wasser/Alkohol-Mischungen löslich sind. Bevorzugt sind Polysaccharide, Polyethylenglycole, Polyacrylsäuren, Polyacrylamide, Polyvinylpyrrolidine und Mischungen dieser Substanzen.

Bevorzugte Polysaccharide sind Chitin, Chitosan und Glucan.

Bevorzugte Polyethylenglykole (PEGs) sind solche mit einem Molekulargewicht von 200 bis 20.000, besonders bevorzugt 500 bis 2.000 und ganz besonders bevorzugt etwa 1.000 g/mol. Ganz besonders bevorzugt ist Polyethylenglycoldimethacrylat mit einem Molekulargewicht von 1.000 g/mol (PEG1000DMA).

Bevorzugte Polyacrylsäuren sind solche mit einem Molekulargewicht von 10.000 bis 60.000 g/mol, besonders bevorzugt 15.000 bis 35.000 g/mol. Ganz besonders bevorzugt ist Polyacrylsäure (PAA) mit einem mittleren Molekulargewicht von etwa 30.000 g/mol, die durch Umsetzung mit 0,5 mol Glycidylmethacrylat (GMA) pro Acrylsäurebaustein im Polymer modifiziert wurde (PAA/GMA). Hierbei handelt es sich um Polyacrylsäure mit einem mittleren Molekulargewicht von 40.000 g/mol, die durch Umsetzung mit 0,5 mol Glycidylmethacrylat pro mol Polymer modifiziert ist.

Als carboxyl- und/oder hydroxylgruppenhaltiges Polymer wird vorzugsweise eine Mischung verschiedener Polymere, besonders bevorzugt eine Mischung von Polyethylenglykol und Polyacrylsäure, ganz besonders bevorzugt eine Mischung der oben definierten bevorzugten Polyethylenglykole und Polyacrylsäuren verwendet.

Säure und carboxyl- und/oder hydroxylgruppenhaltiges Polymer werden vorzugsweise in einem Gewichtsverhältnis von Säure zu Polymer von 1:4 bis 1:8, vorzugsweise 1:5 bis 1:6 eingesetzt.

Zur Behandlung von überempfindlichen Zähnen werden Säure und Polymer miteinander gemischt und auf den zu behandelnden Zahn aufgebracht. Die erfindungsgemäßen Zusammensetzungen sind flüssig. Neben Säure und Polymer enthalten sie vorzugsweise auch ein zur Anwendung im Mundes des Patienten geeignetes Lösungsmittels, vorzugsweise Wasser, Ethanol oder eine Mischung derselben. Bei der Verwendung fester Säuren und Polymere ist die Verwendung eines Lösungsmittels zwingend. Nach dem Auftragen werden der oder die Zähne beispielsweise mit einem Luftstrom getrocknet.

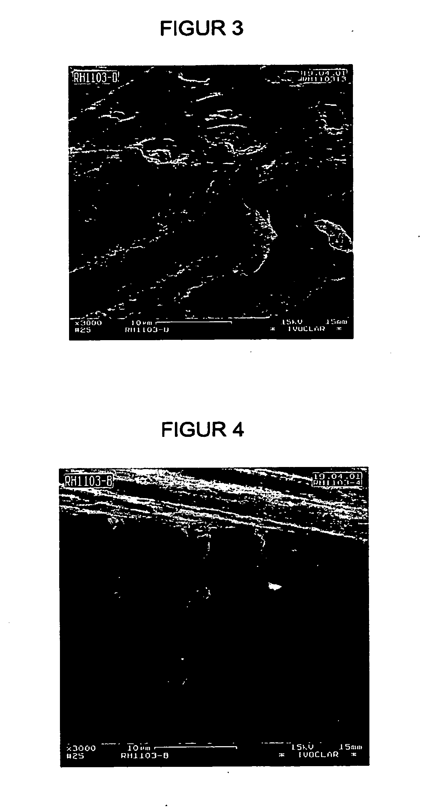

Es wurde gefunden, daß die gleichzeitige Verwendung von Säure und organischen, hydroxyl- und/oder carboxylgruppenhaltigen Polymeren einen praktisch vollständigen Verschluß der Dentintubuli bewirkt. Durch eine spezielle elektronenmikroskopische Aufnahmetechnik konnte gezeigt werden, daß der Verschluß durch weit in die Tubuli hineinreichende Pfropfen erfolgt (Figur 3), die einen sicheren und langanhaltenden Schutz gewährleisten. Dieses Ergebnis ist insofern überraschend als Säuren in der Regel eine sensibilisierende Wirkung zugeschrieben wird.

Die im Stand der Technik bekannten Präparate zur Desensibilisierung von Zähnen ergeben demgegenüber keine durchgehenden, einheitlichen, pfropfförmigen Präzipitate sondern lediglich stegförmige Strukturen (Figur 4), die zwar ebenfalls eine gewisse Barriere gegen Bewegungen des Dentinliquors darstellen, jedoch keine dauerhafte Desensibilisierung der Zähne gewährleisten können.

Es wird angenommen, daß es beim Zusammentreffen der erfindungsgemäß verwendeten Säuren und der erfindungsgemäß verwendeten Polymere mit den Dentinliquorproteinen zur gleichzeitigen und sich möglicherweise gegenseitig bedingenden Fällung von Proteinen, Calcium und Polymer kommt, was zur Ausbildung massiver Pfropfen führt, die durch zusätzliche Reaktion mit den Ca-Anteilen der Tubuliwänden in den Tubuli verankert werden.

Die erfindungsgemäßen Zusammensetzungen enthalten kein Glutaraldehyd und vorzugsweise auch kein Hydroxyethylmethacrylat.

Neben Säure und Polymer können die erfindungsgemäßen Zusammensetzungen zusätzliche Komponenten zur weiteren Verbesserung ihrer Eigenschaften enthalten.

Beispielweise kann durch die Zugabe Kaliumionen freisetzender Verbindungen, vorzugsweise KF, KCl, Kaliumoxalat, K₂SO₄, K₂CO₃, den Kaliumsalzen organischer Verbindungen, z.B. von Polyacrylsäuren und Zuckersäuren, durch eine kurzfristige Erhöhung der extrazellulären Kaliumionenkonzentration ein rascher primärer desensibilisierender Effekt erzielt werden. Eine ähnliche Wirkung kann durch die Zugabe von Strontiumionen erzielt werden, die vorzugsweise in Form von SrCl₂ zugegeben werden.

Durch die Verwendung von Fluoridionen freisetzenden Verbindungen, vorzugsweise NaF, KF, organischen und anorganischen Aminfluoriden, SnF₂ und ZnF₂, läßt sich die Ausfällung von Calcium durch die Bildung von Calciumfluorid begünstigen.

Ein besonders bevorzugter Zusatzstoff ist Kaliumfluorid, das sowohl Kalium- als auch Fluoridionen freizusetzen vermag.

Weiterhin ist die Zugabe von filmbildenden Substanzen vorteilhaft, die einen mechanischen Verschluß der Tubuli bewirken. Hierdurch wird einerseits die Erzielung eines schnellen Primäreffekts begünstigt, andererseits die Zusammensetzung in den Tubuli fixiert und so ein tiefes Eindringen derselben in die Tubuli und das Ausbilden eines festsitzenden Pfropfens gefördert.

Als filmbildenden Substanzen sind Cellulosederivate bevorzugt, insbesondere Celluloseether, wie beispielsweise Hydroxypropylcellulose.

Der pH-Wert der erfindungsgemäßen Zusammensetzungen liegt im Bereich von 1 bis 4, besonders bevorzugt 1,5 bis 3,5 und ganz besonders bevorzugt 2 bis 3. Zur Einstellung und/oder Konstanthaltung des pH-Wert kann es vorteilhaft sein, geeignete Puffersysteme, wie Citrat-, Phosphat-, Phosphonat-, Acetat-, Carbonat-, Sulfonatpuffer, vorzugsweise Phosphonatoder Sulfonatpuffer zuzugeben. Innerhalb des angegebenen pH-Bereichs wird eine gute Konditionierung der Dentinoberfläche erzielt und gleichzeitig die Protein- und Calciumfällung bewirkt.

Bevorzugte Lösungsmittel zur Herstellung der erfindungsgemäßen Zusammensetzungen sind Wasser und Alkohole, wie Methanol, Isopropanol und insbesondere Ethanol, sowie Mischungen von Wasser und Alkohol, besonders bevorzugt Mischungen aus etwa 50 Gew.-% Wasser und etwa 50 Gew.-% Ethanol (bezogen auf die Gesamtmasse des Lösungsmittels) . Mischungen von Wasser und Alkohol enthalten vorzugsweise mindesten 20 Gew.-% Wasser, bezogen auf die Lösungsmittelmasse.

Die genannten Komponenten werden vorzugsweise in den folgenden, unabhängig voneinander wählbaren Mengen eingesetzt:
0,5 bis 40 Gew.-%, vorzugsweise 1,0 bis 10,0 Gew.-% Säure,
1,0 bis 50 Gew.-%, vorzugsweise 5 bis 35 Gew.-% carboxyl- und/oder hydroxylgruppenhaltiges Polymer,
0,5 bis 30 Gew.-%, vorzugsweise 1,0 bis 10 Gew.-% einer filmbildenden Komponente,
0,1 bis 2,0 Gew.-%, vorzugsweise 0,1 bis 1,0 Gew-% Fluorid-Ionen,
0,1 bis 10 Gew.-%, vorzugsweise 0,1 bis 5 Gew.-% Kaliumionen,
0 bis 97,8 Gew.-%, vorzugsweise 40 bis 80 Gew.-% Lösungsmittel.

Bevorzugt sind Zusammensetzungen, bei denen die Mengen aller Komponenten innerhalb der definierten Bereiche liegen.

Die Gesamtmenge an carboxyl- und/oder hydroxylgruppenhaltigem Polymer setzt sich gemäß einer bevorzugten Ausführungsform aus 1,0 bis 40 Gew.-%, vorzugsweise 2,0 bis 10 Gew.-% Polyacrylsäure und
1,0 bis 40 Gew.-%, vorzugsweise 5,0 bis 30 Gew.-% Polyethylenglycoldimethacrylat zusammen.

Darüber hinaus können die Zusammensetzungen 0,1 bis 20 Gew.-% SrCl₂ enthalten.

Ferner können weitere Additive, wie Gingiva schonenden Substanzen, vorzugsweise Dexpanthenol, Chitosan und Hyaluronsäure, und Geschmacksstoffe, beispielsweise Minze, zugesetzt werden. Dexpanthenol wird vorzugsweise in einer Menge von 0 bis 5 Gew.-%, insbesondere 0,5 bis 2,0 Gew.-%, Chitosan und Hyaluronsäure in einer Menge von jeweils 0 bis 20 Gew.-%, insbesondere 0 bis 5 Gew.-% eingesetzt, jeweils bezogen auf die Gesamtmasse der Zusammensetzung. Geschmacksstoffe werden vorzugsweise in einer Menge von 0,1 bis 1,0 Gew-% verwendet.

Ganz besonders bevorzugt sind Zusammensetzungen, die
- 1 bis 5 Gew.-%: Phosphonsäure, insbesondere einer oder mehrerer der oben definierten bevorzugten Phonsphonsäuren,
- 3 bis 7 Gew.-%: Polyacrylsäure,
- 15 bis 25 Gew.-%: Polyethylenglycoldimethacrylat,
- 3 bis 7 Gew.-%: Hydroxypropylcellulose,
- 0,1 bis 1,0 Gew.-%: Kaliumfluorid,
- 0,05 bis 0,2 Gew.-%: Geschmacksstoff und
- 53,8 bis 76,85 Gew.-%: Ethanol/Wasser-Mischung (ca. 50 Gew.- %ig).
enthalten.

Alle Prozentangaben hierin beziehen sich, wenn nicht anders angegeben, auf die Gesamtmasse der Zusammensetzung.

Da die sich die Anwesenheit der Säure nachteilig auf die Haltbarkeit auswirken kann, werden Säure, Polymer und ggf. die übrigen Komponenten der erfindungsgemäßen Zusammensetzungen vorzugsweise in räumlich getrennter Form vertrieben, beispielsweise in Form von Kits. Ein Kit enthält z.B. ein Gefäß mit Säure oder einer Lösung der Säure in einem geeigneten Lösungsmittel, wie Wasser, (Säurekomponente) und ein zweites Gefäß mit dem Polymer und ggf. den übrigen Komponenten oder einer Lösung des Polymers und ggf. der übrigen Komponenten (Polymerkomponente). Alternativ können Mehrkammergefäße, beispielsweise Zweikammergefäße, verwendet werden, die die Säure und die übrige Komponenten in getrennten Kammern enthalten. Die Komponenten der Zusammensetzung können auch auf mehr als zwei Gefäße oder Gefäßkammern verteilt werden. Die Zusammensetzungen von Säurekomponente und Polymerkomponente werden vorzugsweise so bemessen, daß beim Vereinigen der Komponenten die oben definierten Zusammensetzungen erhalten werden.

Besonders bevorzugt sind Kits bei denen die Säure auf einen Pinsel aufgebracht ist. Hierzu wird vorzugsweise eine feste Säure in einem Lösungsmittel gelöst, die Lösung auf einen Pinsel aufgebracht und anschließend das Lösungsmittel verdampft. Vor der Anwendung wird der Pinsel in eine Lösung der übrigen Komponenten eingetaucht und dann der oder die Zähne damit behandelt. Bei dieser Ausführungsform wird die Menge der Lösung der übrigen Bestandteile vorzugsweise auf eine einmalige Anwendung abgestimmt. Größe, Form und Borstenmaterial des Pinsels werden vorzugsweise so gewählt, daß der Pinsel die Menge an Säure aufnimmt, die zusammen mit der zweiten Komponente die gewünschte Zusammensetzung ergibt.

Der Pinsel wird vorzugsweise mit einer Menge von 2 bis 15 mg, besonders bevorzugt 2 bis 8 mg, ganz besonders bevorzugt 2,5 bis 4 mg und ganz insbesondere etwa 3 mg Säure pro Pinsel beladen.

Die Menge der zweiten Komponente beträgt zur einmaligen Anwendung beispielsweise etwa 60 mg, so daß sich mit der Säuremenge des Pinsels eine Gesamtmasse der Zusammensetzung von 62,5 bis 75 mg ergibt. Die Zusammensetzung der zweiten Komponente ist so gewählt, daß nach der Kombination derselben mit der Säure des Pinsels die Gesamtzusammensetzung innerhalb der oben definierten Bereiche liegt. Pinsel und Lösung sind vorzugsweise so in einem Zweikammergefäß untergebracht, daß Pinsel und Flüssigkeit durch einfaches Verschieben des Pinsels miteinander in Kontakt gebracht werden können. Zweikammergefäße diesen Typs werden z.B. in der DE 199 56 705 A1 beschrieben.

Die erfindungsgemäßen Zusammensetzungen eignen sich allgemein zum Fällen von Protein, besonders jedoch zur Desensibilisierung von empfindlichen Zähnen.

Dentintubuli werden häufig auch beim Bearbeiten der Zähne durch den Zahnarzt freigelegt, beispielsweise beim Bohren oder Schleifen der Zähne, was oft mit einer Sensibilisierung verbunden ist. Die erfindungsgemäßen Zusammensetzungen beeinträchtigen die Wirkung üblicher Füllungskomposite nicht und können daher vorteilhaft mit diesen kombiniert werden, d.h. Kavitäten können beispielsweise vor dem Legen der Füllung mit einer erfindungsgemäßen Zusammensetzung behandelt und anschließend mit der Füllung versorgt werden.

Die Erfindung wird im folgenden anhand von Beispielen erläutert.

### Beispiele

### Herstellung von Phosphonsäuren

### Beispiel 1:

### 2-[4-(Dihydroxyphosphoryl)-2-oxa-butyl]-acrylsäure (1)

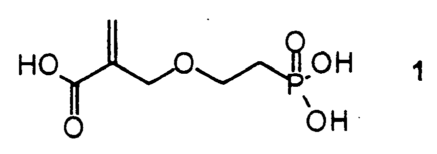

Zu einer Lösung von 66.6 g (0.28 mol) 2-[4-(Dihydroxyphosphoryl)-2-oxa-butyl]-acrylsäureethylester, der durch Umsetzung von 2-Hydroxyethylphosphonsäurediethylester mit α-Chlormethylacrylsäureethylester und nachfolgende Hydrolyse mit Trimethylbromsilan zugänglich ist (vgl. N. Moszner, F. Zeuner, U. K. Fischer, V. Rheinberger, Macromol. Chem. Phys. 200 (1999) 1062), in 50 ml Wasser wurde eine Lösung von 69.6 g (1.74 mol) NaOH in 700 ml Wasser so zugetropft, daß 5 °C nicht überschritten wurden. Nach dem Erwärmen auf Raumtemperatur wurde die Mischung für 16 h gerührt. Die wäßrige Lösung wurde 3-mal mit je 100 ml Methylenchlorid gewaschen und dann unter Rühren und Kühlen (Eisbad) mit 20 %iger Schwefelsäure auf etwa pH 1 gestellt (ca. 500 ml, T < 10 °C) . Ausgefallenes Natriumsulfat wurde abfiltriert und die Lösung nochmals mit 250 ml Methylenchlorid gewaschen. Dann wurde die wäßrige Phase mit Kochsalz gesättigt und 3-mal mit je 250 ml Tetrahydrofuran extrahiert (THF, stabilisiert mit 300 ppm 2, 6-Di-tert. -butyl-p-kresol) . Die vereinigten THF-Lösungen wurden mit Natriumsulfat getrocknet und bis zur Trockene eingeengt. Der erhaltene Kristallbrei wurde erst im Feinvakuum und anschließend über P₂O₅ im Exsikkator nachgetrocknet. Man erhielt 48.8 g (83 %) eines weißen Pulvers, das bei 119 - 120 °C schmilzt.
HPLC: > 99 %
C₆H₁₁O₆P Ber.: C 34.30 H 5.28
(210.12) Gef.: C 34.85 H 5.29
IR: 463 (m), 505 (w), 533 (w), 675 (w), 738 (w), 785 (w), 825 (w), 969 (s; sh), 1027s), 1100 (s; sh), 1172 (m), 1255 (s), 1278 (s), 1370 (w), 1396 (w), 1396 (w), 1427 (w), 1633 (m), 1694 (s), 2304 (m, b), 2924 (s; b)
¹H-NMR (400 MHz, DMSO-d₆, ppm) : 1.82-1. 97 (m; 2H; CH₂-P), 3.56-3. 65 (m; 2H; OCH₂CH₂), 4.15 (s; 2H; CH₂C=C), 5.81 und 6.14 (s, 2x1H, =CH₂); 10.29 (s; 3H; OH). ¹³ C-NMR (100 MHz; DMSO-d₆, ppm): 27. 17 und 28.50 (CH₂P), 65.01 und 67.60 (CH₂OCH₂), 124.43 (CH₂=C), 137.24 (CH₂=C), 167.48 (C=O). ³¹P-NMR (162 MHz, DMSO-d₆, ppm): 23.82

### Beispiel 2:

### 1. Stufe: (cis/trans)-1-Carboxyethyl-2-vinyl-cyclopropan-1-carbonsäure-[2-(dimethoxyphosphoryl)ethyl]ester (2)

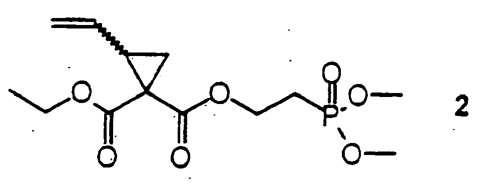

36.8 g (0.2 mol) *(cis*/*trans)*-2-Vinylcyclopropan-1,1-dicarbonsäuremonoethylester, 32 mg Hydrochinonmonomethylether, 732 mg (6.0 mmol) 4-Dimethylamino-pyridin und 30.8 g (0.2 mmol) (2-Hydroxyethyl)-phosphonsäuredimethylester wurden in 800 ml absolutem Methylenchlorid gelöst und auf - 5 °C gekühlt. Zu der leicht trüben Lösung wurden unter Rühren portionsweise 38.4 g (0.2 mol) *N-*(3-Dimethylamino-propyl)*-N'*-ethyl-carbodiimid-hydrochlorid hinzugefügt. Nach dem Erwärmen auf Raumtemperatur wurde die Mischung von 20 h gerührt. Die Lösung wurde je 2-mal mit je 200 ml 2 N Salzsäure, gesättigter Natriumhydrogencarbonatlösung und gesättigter Kochsalzlösung gewaschen und über Natriumsulfat getrocknet. Das Lösungsmittel wurde abdestilliert und das verbleibende farblose Ö1 im Hochvakuum destilliert. Man erhielt 32.2 g (50%) eines farblosen Öls.
HPLC: 95 %
C₁₃H₂₁O₇P Ber.: C 48.75 H 6.61
(320.28) Gef.: C 48.90 H 7.00
IR: 2956 (w, CH₂, CH₃) , 1720 (s, C=O), 1638 (w, C=C), 1447 (m, CH₂, CH₃) , 1370 (m, CH₃) , 1021 (ss, C-O-C) .
¹H-NMR (400 MHz, CDCl₃, ppm) : δ = 1.26 (t; *J* = 7.2 Hz, 3 H, CH₂CH₃), 1.57-1.61, 1.71-1.74 (m; 2 x 1 H, CH₂-cyclopropyl), 2.16-2.25 (m; 2 H, CH₂P), 2.55-2.65 (CH-cyclopropyl), 3.75, 3.78 (s; 2 x 3 H, OCH₃), 4.20 (q; *J* = 7. 0 Hz, OCH₂CH₃), 4.33-4.38 (m; 2 H, OCH₂CH₂P), 5.24 (dd; 2 H, =CH₂), 5.39-5.49 (m; 1 H, =CH) .
¹³C-NMR (CDCl₃, 100 MHz, ppm) : δ = 12.8 (CH₃CH₂), 19.1 (CH₂-cyclopropyl), 23.5 (d, *J*_{c-p} = 141 Hz, CH₂P), 30.0 (CH-cyclopropyl), 34.3 (CO-C-CO), 51.1 (OCH₃), 58.1, 60.0 (2 x OCH₂), 117.4 (=CH₂), 131.5 (=CH), 165.6, 167.8 (2 x C=O).
³¹P-NMR (162 MHz, CDCl₃, ppm): δ = 29.3

### 2. Stufe (cis/trans)-1-Carboxyethyl-2-vinyl-cyclopropan-1-carbonsäure-[2-(dihydroxyphosphoryl)ethyl]ester (3)

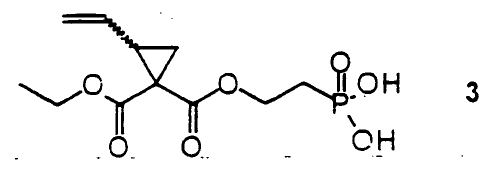

9.61 g (30 mmol) *(cis-trans)*-1-Carboxyethyl-2-vinyl-cyclopropan-1-carbonsäure- [2- (dimethoxyphosphoryl) ethyl] ester 2 wurden in 20 ml absolutem Methylenchlorid gelöst und zu der Lösung 11.5 g (75 mmol) 2.5 Mol-% (9.71 ml) Trimethylbromsilan getropft. Die Mischung wurde für 3 h bei 40 °C reagieren gelassen und dann das Methylenchlorid und überschüssiges Trimethylbromsilan abdestilliert. Der gebildete Silylester wurde mit 35 ml wasserfreiem Methanol versetzt und 6 h bei Raumtemperatur gerührt. Das Methanol wurde wieder abdestilliert und der Rückstand im Feinvakuum getrocknet. Zur Reinigung wird das Rohprodukt in 50 ml Wasser aufgenommen, portionsweise mit 3.15 g (37.5 mmol) Natriumhydrogencarbonat versetzt und 2-mal mit je 25 ml Methylenchlorid gewaschen. Anschließend wurde die wäßrige Phase unter Eiskühlung mit Salzsäure angesäuert und die weiße Emulsion, aus der sich die Phosphonsäure teilweise abscheidet 2-mal mit je 25 ml Methylenchlorid extrahiert. Die vereinigten organischen Phasen wurden mit Natriumsulfat getrocknet, das Methylenchlorid abdestilliert und der Rückstand im Feinvakuum getrocknet. Man erhielt 7.50 g (86 %) eines hochviskosen Öls.
HPLC: 95 %
C₁₁H₁₇O₇P Ber.: C 45.20 H 5.88
(292.25) Gef.: C 44.98 H 5.70
IR: ν = 3200 (sb, OH), 2981 (w, CH₂, CH₃) , 1720 (s, C=O) , 1637 (m, C=C) , 1445 (w, CH₂, CH₃) , 1371 (m, CH₃) , 1195, 1126 (s, C-O-C) , 989, 917 cm⁻¹ (s, =C-H).
¹H-NMR (CDCl₃, 400 MHz, ppm) : δ = 1.26 (t; *J* = 7 Hz, 3 H, CH₃) , 1.63-1.67, 1.75-1.78 (2 m; 2 x 1 H, CH₂-cyclopropyl), 2.17-2.25 (m; 2 H, CH₂P), 2.60-2.65 (m; 1 H, CH-cyclopropyl), 4.20 (q; *J* = 7 Hz, 2 H, CH₂CH₃), 4.31-4.51 (m; 2 H, CH₂CH₂P), 5.14-5.16, 5.29-5.34 (m; 2 x 1 H, =CH₂), 5.41-5.50 (m; 1 H, =CH), 10.56 (br.; 2 H, OH) .
¹³C-NMR (100 MHz, CDCl₃, ppm) : δ = 14.2 (CH₃) , 20.8 (CH₂-cyclopropyl) , 26.2 (d, J_{c-p} = 142 Hz, CH₂P), 32.3 (CH-cyclopropyl) , 35.6 6 (CO-C-CO), 59.9, 61.9 (2x OCH₂), 119.1 (=CH₂), 132.6 (=CH), 167.8, 169.2 (2x C=O), 166.9, 169.9 (C=O, cis-Isomer) ³¹P-NMR (162 MHz, CDCl₃, ppm): 2 Isomere: δ = 29.0 (86 %) + 28.9 (14 % cis-Isomer).

### Beispiel 3:

### 1. Stufe: 5-(Dimethoxyphosphoryl)-2-methylen-4-oxa-5-phenylpentansäureethylester (4)

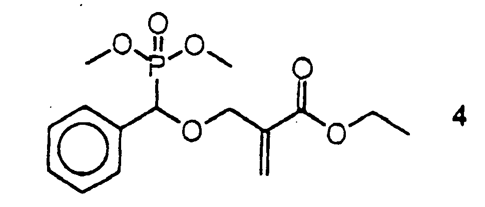

21.6 g (0,1 mol) [(Dimethoxyphosphoryl) -hydroxymethyl]benzol, 10.1 g (0.1 mol) Triethylamin und 0.02 g Phenothiazin wurden unter Argon in 200 ml absolutem THF gelöst und zu der Lösung 14.86 g (0.1 mol) 2-Chlormethylacrylsäureethylester getropft. Die Mischung wurde für 15 h bei 60 - 64 °C reagieren gelassen, auf Raumtemperatur abgekühlt und vom ausgefallenen Triethylaminhydrochlorid abgesaugt. Der Niederschlag wurde mit Diethylether gewaschen und der Waschether mit dem Filtrat vereinigt. Die vereinigten organischen Lösungen wurden anschließend mit 400 ml Wasser gewaschen und das Waschwasser 3 mal mit je 100 ml Diethylether reextrahiert. Die organischen Lösungen wurden erneut vereinigt, mit 100 ml gesättigter Kochsalzlösung gewaschen und über Natriumsulfat getrocknet. Nach Einengen am Rotationsverdampfer und Entfernen des restlichen THF erhielt man 23 g eines farblosen flüssigen Rohprodukts. Zur weiteren Reinigung wurde im Hochvakuum, unter Zusatz von 0.1 g Phenothiazin, destilliert, und man erhielt 15.9 g (48 %) eines farblosen viskosen Öls.
C₁₅H₂₁O₆P Ber.: C 54.88 H 6.45
(328.30) Gef.: C 54.70 H 6.23
IR: ν = 465 (s,b), 701 (m), 771 (w), 832 (m), 1031 (s, sh), 1095 (s), 1181 (s), 1262 (s), 1309 (m), 14001 (w), 1453 (m), 1637 (w), 1718 (s), 2854 (w), 2956 (m).
¹H-NMR (400 MHz, CDCl₃, ppm):1.29 (t; 3H, CH₃CH₂), 3.65-3.73 (2d; 6 H, CH₃O), 4.14-4.33 (m; 4H, CH₂) , 4.78 (d; 1H, CH) , 5.97 und 6.34 (s; 2 x 1H, CH₂=) , 7.34-7.47 (m; 5H, CH-arom).
¹³C-NMR (100 MHz, CDCl₃, ppm) : 14.5 (s, CH₃-CH₂); 53.8 und 54.2 (s, 2 x CH₃O), 61.1 (OCH₂CH₃), 69.88 (s, OCH₂C=CH₂), 78.5 (d, *J*_{c-p} = 168 Hz, CHP); 126.9 (s, CH₂=); 128.3 und 128.9 (s, CH-arom.); 135.0 und 136.8 (CH₂=C und C-arom.), 165.4 (C=O).
³¹P (162 MHz, CDCl₃, ppm) :21.30 (s)

### 2. Stufe: 5-(Dihydroxyphosphoryl)-2-methylen-4-oxa-5-phenylpentansäureethylester (5)

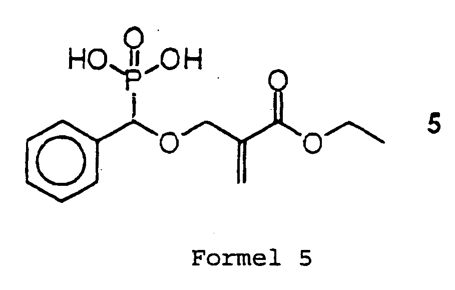

14.4 g (0.044 mol) 5-(Dimethoxyphosphoryl)-2-methylen-4-oxa-5-phenylpentansäureethylester 4 wurden analog Beispiel 2 (2. Stufe) mit 17.2 g (0.11 mol) Trimethylsilylbromid in 40 ml Methylenchlorid umgesetzt und aufgearbeitet. Man erhielt 10.0 g (76 %) eines weißen Pulvers.
C₁₃H₁₇O₆P Ber.: C 52.00 H 5.71
(300.25) Gef.: C 52.15 H 5.67
IR: ν = 698 (s), 739 (w), 805 (w), 819 (w), 970 (s), 1026 (s, sh), 1094 (s, sh), 1178 (s, sh), 1280 (s, sh), 1320 (m), 1340 (m), 1402 (m, sh), 1453 (m, sh), 1490 (m), 1634 (m), 1713 (s), 2321 (m), 2910 (m), 2949 (m), 2982 (m).
¹H-NMR (400 MHz, CDCl₃, ppm): 1.22 (t; 3H, CH₃) , 4.06 - 4.17 (m, 4H, CH₂) , 4.50 (d; *J* = 4 Hz, 2H, CHP), 5.89 und 6.19 (s, 2 x 1H, CH₂=), 7.18 - 7.33 (m, 5H, CH-arom.); 10.79 (s; 2H, OH, H/D-Aust.).
¹³C-NMR (100 MHz, CDCl₃, ppm) :14.1 (CH₃) , 60.7 (OCH₂CH₃, 68.5 (CH₂OCH), 78.0 (d, *J*_{c-p} = 166.5 Hz, CH-P) , 127.5 - 128.3, 135.2, 136.2 (alle C-arom. + CH₂=C), 166.1 (C=O).
³¹P-NMR (162 MHz, CDCl₃, ppm) :20.1.

### Herstellung und Untersuchung von Desensibilisierungsmitteln

### Beispiel 4:

### Herstellung eines einkomponentigen Desensibilisierungsmittels

Durch Mischen der Komponenten wurde ein Desensibilisierungsmittel folgender Zusammensetzung hergestellt:

| Komponente | Anteil [Gew.-%] |
|---|---|
| Phosphonsäure aus Bsp. 1 | 4,0 |
| Polyacrylsäure (PAA/GMA) | 5,0 |
| Polyethylenglykol (PEG 1000 DMA) | 20 |
| Hydroxypropylcellulose | 5,0 |
| Kaliumfluorid | 0,3 |
| Geschmacksstoff (Optamint) | 0,1 |
| Ethanol/Wasser (50:50) | 65,6 |

### Beispiel 5:

### Herstellung eines zweikomponentigen Desensibilisierungsmittels

Es wurde eine 20 Gew.-%ige Lösung der Phosphonsäure aus Beispiel 1 in Ethanol hergestellt. In diese Lösung wurden kleine Pinsel getaucht und anschließend getrocknet. Dieser Vorgang wurde so oft wiederholt, bis die Säuremenge nach dem Trocknen 3,0 ± 0,3 mg pro Pinsel betrug.

Als zweite Komponente wurde eine Mischung folgender Zusammensetzung hergestellt:

| Komponente | Anteil [g] |
|---|---|
| Polyacrylsäure (PAA/GMA) | 5,0 |
| Polyethylenglykol (PEG 1000 DMA) | 20 |
| Hydroxypropylcellulose | 5,0 |
| Kaliumfluorid | 0,3 |
| Geschmacksstoff (Optamint) | 0,1 |
| Ethanol/Wasser (50:50) | 65,6 |

Die zweite Komponente wurde in Portionen von jeweils 60 mg aufgeteilt. Anschließend wurde jeweils ein Pinsel in eine 60 mg Portion der zweiten Komponente eingetaucht und mit dem Pinsel durchmischt. Es wird eine theoretische Säurekonzentration in der Zusammensetzung von etwa 4,8 Gew.-% erreicht.

### Beispiel 6:

### Messung der Proteinfällung

Zur Bestimmung der proteinfällenden Eigenschaften der erfindungsgemäßen Zusammensetzungen wurde Pferdeserum verwendet (Horse Serum, PAA Laboratories GmbH, Linz, Österreich, Kat.-Nr. B15-021) das mit physiologischer Kochsalzlösung (8,5 g NaCl pro 1 1 H₂O) verdünnt wurde (1 Volumenteil Pferdeserum + 2 Volumenteile Kochsalzlösung), um eine mit Dentinliquor vergleichbare Proteinkonzentration zu erhalten. Die verdünnte Proteinlösung wurde weiterhin mit CaCl₂ versetzt (2 mM), um die Calciumionenkonzentration an die von Dentinliquor anzugleichen.

500 µl der Proteinlösung wurden anschließend mit 500 µm der zu testenden Zusammensetzung versetzt und die Mischung für 30 Minuten bei Raumtemperatur stehengelassen, zentrifugiert und die erhaltenen Präzipitate bei 75 °C getrocknet und gewogen.

Zur Proteinfällung wurde die in Beispiel 5 beschriebenen Lösung (2. Komponente) verwendet, die mit Phosphonsäure in Kozentrationen von 10 mM bis 200 mM versetzt wurde (vgl. Tabelle 1) . Pro Konzentration wurden 5 Fällungen durchgeführt und anschließend der Mittelwert der Bestimmungen berechnet. Die Ergebnisse sind in der Tabelle 1 angegeben.

Diese Ergebnisse zeigen, daß bei den erfindungsgemäßen Zusammensetzungen schon sehr geringe Säurekonzentrationen eine nennenswerte Proteinfällung induzieren, bereits bei 150 mM Säure ist die Fällung quantitativ.

Auf die oben beschriebene Weise wurden weitere erfindungsgemäße Zusammensetzungen getestet . Die Zusammensetzungen der Proben und die Ergebnisse sind in Tabelle 2 zusammengefaßt.

**Tabelle 1**

| **Einfluß der Säurekonzentration auf die Proteinfällung** | |
|---|---|
| **Säurekonzentration** **[mM]** | **Präzipität¹** **[mg]** |
| 10 | 55,3 |
| 25 | 58.2 |
| 50 | 58,8 |
| 75 | 69,1 |
| 100 | 70,8 |
| 150 | 74,2 |
| 200 | 72.3 |

| | |
|---|---|
| ¹ Mittelwert von 5 Messungen | |

**Tabelle 2**

| **Proteinfällung erfindungsgemäßer Zusammensetzungen** | | | | | | | | |
|---|---|---|---|---|---|---|---|---|
| **Probe** **Nr.** | **Zusammensetzung (Angaben in Gew.-%, Differenz zu 100 %:H₂O)** | | | | | | | **Pellet** **[mg]** |
| | **Säure** | | **Polymer** | | **KF⁵** | **HPC⁶** | **EtOH** | |
| | P-Säure¹ | SSA² | PEG1000-DMA³ | PO-25⁴ | | | | |
| 1 | 1,0 | - | 9,0 | - | - | - | - | 24,9 |
| 2 | 1,8 | - | 5,5 | 9,1 | - | - | 4,5 | 42,8 |
| 3 | 2,0 | - | 8,9 | 2,5 | - | - | 0,05 | 57,5 |
| 4 | 1,0 | - | 8,9 | 2,5 | - | 0,25 | 2,15 | 59,3 |
| 5 | - | 2,5 | - | 3,0 | - | - | - | 45,2 |
| 6 | - | 1,4 | 5,5 | 1,4 | - | - | 18,2 | 31,9 |
| 7 | - | 1,3 | 8,9 | 2,5 | 0,05 | - | - | 55,7 |
| 8 | - | 0,6 | 8,9 | 2,5 | 0,05 | 0,25 | 2,1 | 57,4 |

| | | | | | | | | |
|---|---|---|---|---|---|---|---|---|
| ¹ Phosphonsäure aus Beispiel 1 ² Sulfosalicylsäure ³ Polyethylenglycoldimethacrylat, Molekulargewicht 1000 g/mol ⁴ Polyacrylsäure, mittleres Mw 40.000 g/mol, modifiziert mit 0,5 mol Glycidylmethacrylat/mol Polyacrylsäure ⁵ Kaliumfluorid ⁶ Hydroxypropylcellulose | | | | | | | | |

### Beispiel 7:

### Elektronenmikroskopische Untersuchung der Wirkung von Desensibilisierungsmitteln auf Rinderzähne

Um die Wirkung von Desensibilisierungsmitteln auf natürliche Zähne zu untersuchen, wurden diese auf Rinderzähne appliziert. Hierzu wurden die Zähne in Kunstharz eingebettet und mit einer Schleifscheibe mit Siliciumcarbid-Schleifpapier (Grit 120/1000) auf die oberste Dentinschicht abgeschliffen. Anschließend wurden die Zähne aus dem Kunstharz herausgebrochen und mit Wasser abgespült. Danach wurde der Pulpen-Raum mit einem handelsüblichen Dentallack (Heliobond, Firma Ivoclar Vivadent AG) versiegelt. Die Zähne wurden dann einer Säureätzung unterworfen, indem das Dentin für 15 Sekunden mit einem handelsüblichen dentalen Ätzgel (37% Phosphorsäure, Email-Preparator blau, Firma Ivoclar Vivadent AG) behandelt wurde. Nach Spülen mit Wasser und Trocknen wurde die zu untersuchende Zusammensetzung aufgebracht und für 30 Sekunden auf den Zähnen belassen. Danach wurden die Zähne erneut mit Wasser gespült, dann für 6 Stunden bei 37 °C in 0,85 % NaCl/2 mM CaCl₂-Lösung gelagert und anschließend für 4 Tage bei 75 °C getrocknet.

Zur elektronenmikroskopischen Untersuchung der Zähne wurden diese gebrochen und sowohl die Dentinoberfläche als auch die Bruchkanten untersucht.

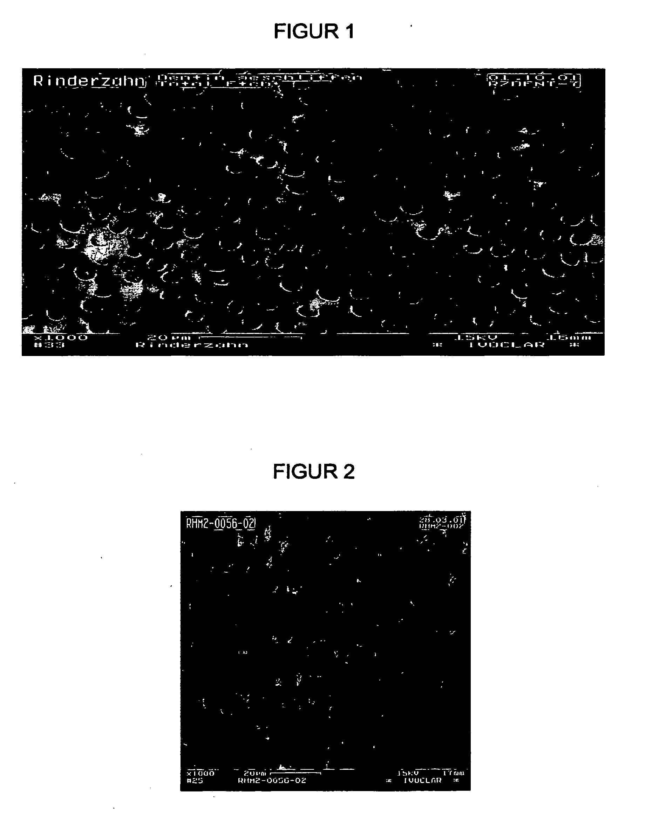

**Figur 1** zeigt die Oberfläche eines beschliffenen und geätzten Zahns, der nicht mit Desensibilisierungsmittel behandelt wurde. Deutlich sind die Dentintubili zu sehen.

**Figur 2** zeigt die Oberfläche eines Zahns, der mit dem in Beispiel 4 beschriebenen Desensibilisierungsmittel behandelt wurde. Die Vergrößerung entspricht der von Figur 1 (1000fach). Die Dentintubuli sind hier verschlossen. Die in Figur 2 erkennbaren Spalten in den Tubulipfropfen sind auf das extreme, für die REM-Aufnahmen notwendige Trocknen der Zähne zurückzuführen.

**Figur 3** ist eine Aufnahme entlang der Bruckkante eines Zahns (3000fach). Deutlich sind sowohl die Zahnoberfläche mit den verschlossenen Tubuliöffnungen als auch angebrochene Tubuli zu erkennen. Die Bruchtechnik ermöglicht Einblick in die Tubuli und zeigt, daß die Verschlußpfropfen weit, d.h. mindestens etwa 20 µm in die Tubuli hineinreichen. Die Spalte zwischen Pfropfen und Tubuliwand sind wiederum auf das Trocknen der Zähne und das damit verbundene Schrumpfen zurückzuführen.

Zum Vergleich wurde ein Zahn auf die oben beschriebene Weise mit einem Desensibilisierungsmittel gemäß dem Stand der Technik behandelt (Gluma, Firma Heraeus Kulzer). Die in Figur 4 gezeigte elektronenmikroskopische Aufnahme der Bruckante des Zahns (Vergrößerung 3000fach) läßt deutlich die Unterschiede zu den erfindungsgemäßen Produkten erkennen. Anders als bei den erfindungsgemäßen Zusammensetzungen werden keine durchgehenden, einheitlichen Präzipitate, sondern lediglich Querverbindungen und kurze Pfropfen gebildet.

### Beispiel 8:

### Untersuchung der Wirkung von Desensibilisierungsmitteln auf menschliche Zähne durch Laser-Scanning-Mikroskopie

Zur Behandlung menschlicher Molaren wurde das in Beispiel 5 beschriebene zweikomponentige Material verwendet. Um eine Untersuchung der Zähne durch Laser-Scanning-Mikroskopie zu ermöglichen, wurde mit einem Pinsel zunächst eine kleine Menge Fluorescein aufgenommen und durch mehrfaches Eintauchen (maximal sechsmal) mit der flüssigen Komponente des Desensibilisierungsmittels vermischt. Sofort im Anschluß wurde das Produkt auf grob gereinigte menschliche Molaren aufgetragen und diese anschließend mit Wasser gespült. Danach wurde mit einer sich langsam bewegenden Säge eine Scheibe aus jedem Zahn herausgeschnitten und mit einem konfokalen Laser-Scanning-Mikroskop untersucht.

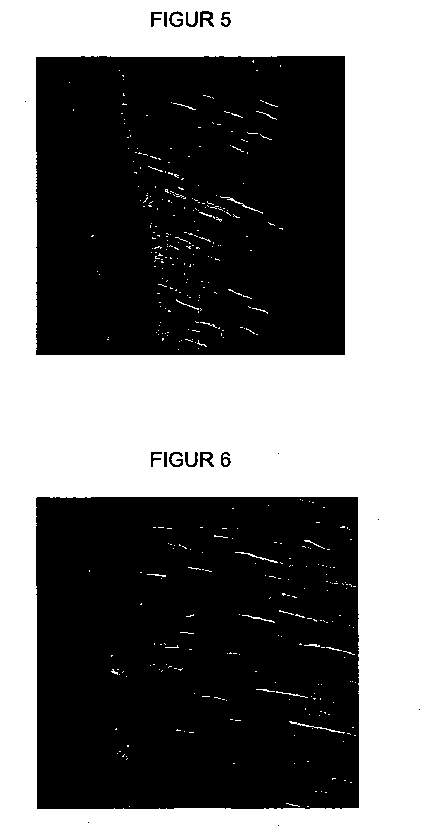

**Figur 5** zeigt eine Aufnahme einer solchen Scheibe. An dem grünen Rand ist deutlich die Filmbildung sowohl auf dem Dentin als auch auf dem Enamel des Zahns zu erkennen. Die grünen Striche zeigen ein tiefes Eindringen des Desensibilisierungsmittels in die Tubuli. Es wurde ein Eindringen von mehr als 200 µm beobachtet.

Um den natürlichen Dentinliquordruck zu simulieren wurde in einem zweiten Experiment die in Beispiel 6 beschriebene Pferdeserumlösung durch die Pulpakammer in die Tubuli geleitet und unter einem hydrostatischen Druck des simulierten Dentinliquors von 60 cm Flüssigkeitssäule das Desensibilisierungsmittel aufgetragen. Es konnte nachgewiesen werden, daß das Desensibilisierungsmittel selbst unter diesen Bedingungen noch 40 bis 50 µm in die Tubuli eindringt (Figur 6, Bereiche mit Desensibilisierungsmittel erscheinen grün, Bereiche ohne Desensibilisierungsmittel rot).

### Beispiel 9

### Kombination von Desensibilisierungsmitteln mit Füllungskompositen

Das legen von Füllungen erfordert in - der Regel zunächst ein Abtragen erkrankter Zahnsubstanz, was mit einem Freilegen von Dentintubuli und einer Sensibilisierung des Zahns verbunden ist. Es wurde daher untersucht, inwieweit erfindungsgemäße Desensibilisierungsmittel mit herkömmlichen Füllungskompositen kompatibel sind.

Rinderzähne wurden zunächst für 15 Sekunden mit einem handelsüblichen Ätzgel (37% Phosphorsäure, Email Preparator blau, Firma Ivoclar Vivadent AG) angeätzt und dann mit Wasser abgespült. Anschließend wurde das in Beispiel 4 beschriebene Desensibilisierungsmittel appliziert (für 5 Sekunden) und für 30 Sekunden einwirken gelassen. Nach dem erneuten Spülen mit Wasser wurde im Luftstrom getrocknet, ein handelsüblicher Haftvermittler (Excite, Firma Ivoclar Vivadent AG) aufgetragen, für 10 Sekunden einwirken gelassen, erneut im Luftstrom getrocknet und der Zahn dann mit Licht einer Wellenlänge von 400 bis 510 nm (Halogenlicht-Polymerisationsgerät, Typ Astralis 7, Firma Ivoclar Vivadent AG) bei 750 mW/cm² für 20 Sekunden belichtet. Schließlich wurden 2 Schichten eines handelsüblichen Füllungskomposits (Tetric Ceram, Fa. Ivoclar Vivadent AG) aufgetragen und jeweils durch Belichten für 40 Sekunden wie oben beschrieben gehärtet.

Anschließend wurde die Scherhaftfestigkeit auf übliche Weise gemessen. Diese betrug 30 MPa, während sich bei gleich behandelten Zähnen ohne Desensibilisierungsmittel ein Wert von 29,5 MPa ergab. Dieser Unterschied ist statistisch nicht signifikant. Somit stellt das Desensibilisierungsmittel keine Beeinträchtigung des Adhäsionsprozesses dar.

## Patentansprüche

1. Dentalzusammensetzung, **dadurch gekennzeichnet, daß** sie eine Säure mit protein- und calciumfällenden Eigenschaften und ein organisches Polymer enthält, das Carboxyl- und/oder Hydroxylgruppen aufweist, und einen pH-Wert im Bereich von 1 bis 3,5 hat.

2. Zusammensetzung nach Anspruch 1, **dadurch gekennzeichnet, daß** sie eine Säure enthält, die in Wasser oder in einer Mischung aus 50 Gew.-% Wasser und 50 Ges.-% Ethanol eine Löslichkeit von 0,5 bis 20"Gew.-% aufweist.

3. Zusammensetzung nach einem der Ansprüche 1 bis 2, **dadurch gekennzeichnet, daß** sie als Säure eine Carbonsäure, Sulfonsäure und/oder Phosphonsäure enthält.

4. Zusammensetzung nach Anspruch 3, **dadurch gekennzeichnet, daß** sie eine Phosphonsäure mit der Formel 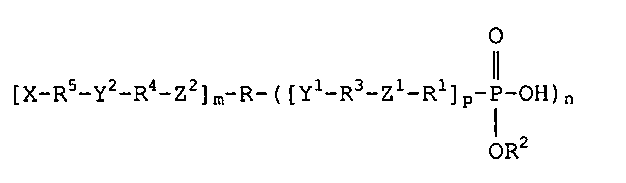 in der
n 1, 2, 3 oder 4 ist,
m 0, 1 oder 2 ist,
p 0 oder 1 ist,
R ein geradkettiger oder verzweigter aliphatischer Kohlenwasserstoffrest mit 1 bis 12 Kohlenstoffatomen oder ein aromatischer Kohlenwasserstoffrest mit 6 bis 12 Kohlenstoffatomen oder ein aliphatisch/aromatischer Kohlenwasserstoffrest mit 7 bis 16 Kohlenstoffatomen ist, der durch OH, NH₂ und/oder COOR⁶ substituiert sein kann,
R¹ ein C₁- bis C₁₂-Alkylen-, C₄- bis C₁₂ Cycloalkylen-, C₆- bis C₁₂-Arylen- oder C₇- bis C₁₆-Alkylenarylenrest ist, der durch OH, NH₂ und/oder COOR⁶ substituiert sein kann, oder entfällt,
R² H, ein C₁- bis C₆-Alkyl- oder ein Phenylrest ist,
R³, R⁴ unabhängig voneinander jeweils einen C₁- bis C₁₂-Alkylen-, C₆- bis C₁₂-Arylen- oder C₇- bis C₁₆-Alkylenarylenrest bedeuten, der durch Methyl, Phenyl oder Fluor substituiert sein kann, oder entfallen,
R⁵ -CH=CR¹³-, ein Prop-1-en-1, 3-diyl-, C₁- bis C₆-Alkenylen-, C₃- bis C₉-Cycloalkylen-, C₁- bis C₆-Alkylen- oder Phenylenrest oder eine Gruppe mit der Formel 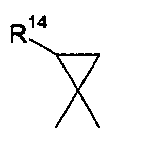 ist,
R⁶ H, ein C₁- bis C₆-Alkyl- oder ein Phenylrest ist,
Z¹,Z² unabhängig voneinander jeweils CO-O, CO-NR⁷, O-CO-NH, O, NH, S bedeuten oder entfallen,
Y¹,Y² unabhängig voneinander jeweils O, CO-O, CO-NR⁸, O-CO-NH bedeuten oder entfallen,
R⁷,R⁸ unabhängig voneinander jeweils H, oder einen C₁-bis C₆-Alkylrest bedeuten,
X H, CN, N(R⁹)₂, OR¹⁰, COOR¹¹ oder CONR₂¹² ist,
R⁹,R¹⁰,R¹¹,R¹² unabhängig voneinander jeweils H, einen C₁-bis C₁₀-Alkyl- oder einen Phenylrest bedeuten,
R13 H oder ein Methylrest ist,
R14 H oder ein C₁- bis C₁₀-Alkyl-, Vinyl- oder Phenylrest ist
enthält.

5. Zusammensetzung nach Anspruch 4, **dadurch gekennzeichnet, daß**
n 1 oder 2 ist und/oder
m 1 ist und/oder
p 0 ist und/oder
R ein aliphatischer geradkettiger oder verzweigter ein- bis fünf-wertiger Alkanrest mit 1 bis 7 Kohlenstoffatomen, ein aromatischer Kohlenwasserstoffrest mit 6 Kohlenstoffatomen oder ein aliphatisch/aromatischer Kohlenwasserstoffrest mit 8 Kohlenstoffatomen ist und/oder
R¹ ein Methylen- oder Ethylenrest ist oder entfällt und/oder
R² H, ein Methyl- oder Ethylrest ist und/oder
R³,R⁴ unabhängig voneinander jeweils einen Methylen-, Ethylen-, Trimethylen-, p-Phenylen-, Ethyliden-, 1-Methylenethan-1,2-diylrest bedeuten oder entfallen und/oder
R⁵ ein Methylen-, Ethylen-, Trimethylen-, Ethen-1,2-diyl-, Methylethylen-, Prop-1-en-1,3-diyl-, oder ein in 2-Stellung einfach substituierter Cyclopropylidenrest ist oder entfällt und/oder
R6 H ist und/oder
Z¹,Z² unabhängig voneinander jeweils CO-O, O-CO-NH oder O bedeuten oder entfallen und/oder
Y¹,Y² unabhängig voneinander jeweils O, CO-O oder CO-NR⁸ bedeuten oder entfallen und/oder
R⁷,R⁸ unabhängig voneinander jeweils H oder einen Methyl- oder Ethylrest bedeuten und/oder
X H, CN, COOR¹¹ oder CONR₂¹² ist und/oder
R⁹,R¹⁰,R¹¹,R¹² unabhängig voneinander jeweils H oder einen Methyl-, Ethyl- oder Phenylrest bedeuten und/oder
R13 H oder ein Methylrest ist,
R14 H oder ein Vinyl- oder Phenylrest ist.

6. Zusammensetzung nach Anspruch 4, **dadurch gekennzeichnet, daß**
n 1 ist,
m 1 ist,
p 0 ist,
R ein C₁- bis C₃-Alkylen- oder Phenylenrest ist,
R2 H ist,
R⁴ ein verzweigter oder geradkettiger C₁- bis C₆-Alkylenrest ist, der durch 1 bis 2 Fluoratome und/oder 1 Phenylrest substituiert sein kann oder entfällt,
R⁵ ein 1-Methylenethan-1,2-diylrest ist,
Z² entfällt,
Y2 O ist oder entfällt,
X COOR¹¹ ist und
R¹¹ H oder ein C₁- bis C₅-Alkyl- oder Phenylrest ist.

7. Zusammensetzung nach Anspruch 4, **dadurch gekennzeichnet, daß**
n 2 ist,
m 2 ist,
p 1 ist,
R ein vierwertiger aliphatischer, aromatischer, oder aliphatisch-aromatischer Kohlenwasserstoffrest mit 2 bis 12 Kohlenstoffatomen ist,
R¹ entfällt,
R2 H ist,
R³ ein C₁- bis C₃-Alkylen- oder Phenylenrest ist oder entfällt,
R⁴ ein verzweigter oder geradkettiger C₁- bis C₆-Alkylenrest ist, der durch 1 bis 2 Fluoratome und/oder 1 Phenylrest substituiert sein kann oder entfällt,
R⁵ ein 1-Methylenethan-1,2-diylrest ist,
Z¹,Z² entfallen,
Y¹ entfällt,
Y2 O ist oder entfällt,
X COOOR¹¹ ist und
R¹¹ H oder ein C₁- bis C₅-Alkyl- oder Phenylrest ist.

8. Zusammensetzung nach einem der Ansprüche 3 bis 7, **dadurch gekennzeichnet, daß** sie als Carbonsäure Maleinsäure und/- oder Trichloressigsäure enthält.

9. Zusammensetzung nach einem der Ansprüche 3 bis 8, **dadurch gekennzeichnet, daß** sie als Sulfonsäure Sulfosalicylsäure (2-Hydroxy-5-sulfobenzoesäure) enthält.

10. Zusammensetzung nach einem der Ansprüche 1 bis 9, **dadurch gekennzeichnet, daß** sie 1 bis 4 unterschiedliche Säuren enthält.

11. Zusammensetzung nach einem der Ansprüche 1 bis 10, **dadurch gekennzeichnet, daß** sie als Polymer ein Polysaccharid, ein Polyethylenglycol, eine Polyacrylsäure, ein Polyacrylamid, ein Polyvinylpyrrolidin oder eine Mischungen dieser Substanzen enthält.

12. Zusammensetzung nach Anspruch 11, **dadurch gekennzeichnet, daß** sie als Polymer eine Mischung aus Polyethylenglycoldimethacrylat und Polyacrylsäure enthält.

13. Zusammensetzung nach einem der Ansprüche 1 bis. 12, **dadurch gekennzeichnet, daß** sie weiterhin Fluoridionen enthält.

14. Zusammensetzung nach einem der Ansprüche 1 bis 13, **dadurch gekennzeichnet, daß** sie weiterhin eine Kaliumionen freisetzende Verbindung enthält.

15. Zusammensetzung nach einem der Ansprüche 1 bis 14, **dadurch gekennzeichnet, daß** sie weiterhin eine filmbildende Komponente enthält.

16. Zusammensetzung nach Anspruch 15, **dadurch gekennzeichnet, daß** sie Hydroxypropylcellulose enthält.

17. Zusammensetzung nach einem der Ansprüche 1 bis 16, **dadurch gekennzeichnet, daß** sie
| | |
|---|---|
| 0,5 bis 40 Gew.-% | Phosphonsäure und/oder |
| 1,0 bis 40 Gew.-% | carboxyl- und/oder hydroxylgruppenhaltiges Polymer und/oder |
| 0,5 bis 30 Gew.-% | einer filmbildenden Komponente und/-oder |
| 0,1 bis 1,0 Gew.-% | Fluorid-Ionen und/oder |
| 0,1 bis 10 Gew.-% | Kaliumionen und |
| 0 bis 97,8 Gew.-% | Lösungsmittel |
enthält.

18. Zusammensetzung nach Anspruch 17, **dadurch gekennzeichnet, daß** sie zusätzlich 0,1 bis 1,0 Gew-% Geschmacksstoffe enthält.

19. Zusammensetzung nach Anspruch 17 oder 18, **dadurch gekennzeichnet, daß** sie als Lösungsmittel ein Gemisch aus Ethanol und Wasser enthält.

20. Zusammensetzung nach einem der Ansprüche 17 bis 19, **dadurch gekennzeichnet, daß** sie
| | |
|---|---|
| 1 bis 5 Gew.-% | mindestens einer Phosphonsäure, |
| 3 bis 7 Gew.-% | Polyacrylsäure, |
| 15 bis 25 Gew.-% | Polyethylenglycoldimethacrylat, |
| 3 bis 7 Gew.-% | Hydroxypropylcellulose, |
| 0,1 bis 1,0 Gew.-% | Kaliumfluorid, |
| 0,05 bis 0,2 Gew.-% | Geschmacksstoff und |
| 53,8 bis 76,9 Gew.-% | Ethanol/Wasser-Mischung (ca. 50 Gew.-%ig) |
enthält.

21. Kit zur Herstellung einer Dentalzusammensetzung gemäß einem der Ansprüche 1 bis 20 enthaltend eine Säure mit protein- und calciumfällenden Eigenschaften und in räumlich getrennter Form davon ein organisches, carboxyl- und/oder hydroxylgruppenhaltiges Polymer.

22. Kit nach Anspruch 21, **dadurch gekennzeichnet, daß** die Säure auf einen Pinsel aufgebracht ist.

23. Kit nach Anspruch 21 oder 22, **dadurch.gekennzeichnet, daß** er eine Lösung des Polymers enthält, deren Zusammensetzung so bemessen ist, daß bei der Kombination der Lösung mit der Säure des Kits eine Zusammensetzung gemäß einem der Ansprüche 18 bis 21 erhalten wird.

24. Kit nach einem der Ansprüche 21 bis 23, **dadurch gekennzeichnet, daß** Säure und Polymer in unterschiedlichen Kammern eines Zweikammergefäßes untergebracht sind.

25. Verwendung einer wie in den Ansprüchen 1 bis 20 definierten Zusammensetzung zum Fällen von Protein.

26. Verwendung einer wie in den Ansprüchen 1 bis 20 definierten Zusammensetzung zur Desensibilisierung von Zähnen.

27. Verwendung einer Zusammensetzung gemäß einem der Ansprüche 1 bis 20 zur Herstellung eines Mittels zur Desensibilisierung von Zähnen.

## Claims

1. Dental composition comprising an acid with protein- and/or calcium-precipitating properties and an organic polymer that has carboxyl and/or hydroxyl groups, and a pH in the range 1 to 3.5.

2. Composition according to claim 1 comprising an acid that has a solubility of 0.5 to 20 wt.% in water or in a mixture of 50 wt.% water and 50 wt.% ethanol.

3. Composition according to claim 1 or 2 comprising a carboxylic acid, sulfonic acid and/or phosphonic acid as the acid.

4. Composition according to claim 4 comprising a phosphonic acid of formula 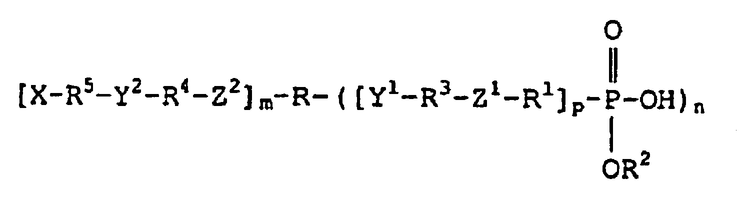 in which
n is 1, 2, 3 or 4,
m is 0, 1 or 2,
p is 0 or 1,
R is a straight-chain or branched aliphatic hydrocarbon group containing 1 to 12 carbon atoms or an aromatic hydrocarbon group containing 6 to 12 carbon atoms or an aliphatic/aromatic hydrocarbon group containing 7 to 16 carbon atoms, which can be substituted by OH, NH₂ and/or COOR⁶,
R¹ is a C₁ to C₁₂ alkylene, C₄ to C₁₂ cycloalkylene, C₆ to C₁₂ arylene or C₇ to C₁₆ alkylene-arylene group, which can be substituted by OH, NH₂ and/or COOR⁶, or is absent,
R² is H, a C₁ to C₆ alkyl or a phenyl group,
R³, R⁴ each mean, independently of each other, a C₁ to C₁₂ alkylene, C₆ to C₁₂ arylene or C₇ to C₁₆ alkylene-arylene group, which can be substituted by methyl, phenyl or fluorine, or are absent,
R⁵ is -CH-CR¹³-, a prop-1-ene-1,3-diyl, C₁ to C₆ alkenylene, C₃ to C₉ cycloalkylene, C₁ to C₆ alkylene or phenylene group or a group of formula 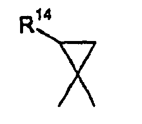
R⁶ is H, a C₁ to C₆ alkyl or a phenyl group,
Z¹, Z² each mean, independently of each other, CO-O, CO-NR⁷, O-CO-NH, O, NH, S or are absent,
Y¹, Y² each mean, independently of each other, O, CO-O, CO-NR⁸, O-CO-NH or are absent,
R⁷, R⁸ each mean, independently of each other, H, or a C₁ to C₆ alkyl group,
X is H, CN, N(R⁹)₂, OR¹⁰, COOR¹¹ or CONR₂¹²,
R⁹, R¹⁰, R¹¹, R¹² each mean, independently of each other, H, a C₁ to C₁₀ alkyl or a phenyl group,
R¹³ is H or a methyl group,
R¹⁴ is H or a C₁ to C₁₀ alkyl, vinyl or phenyl group.

5. Composition according to claim 4 **characterised in that**
n is 1 or 2 and/or
m is 1 and/or
p is 0 and/or
R is an aliphatic straight-chained or branched mono- to pentavalent alkane group containing 1 to 7 carbon atoms, an aromatic hydrocarbon group with 6 carbon atoms or an aliphatic/aromatic hydrocarbon group with 8 carbon atoms and/or
R¹ is a methylene or ethylene group or is absent and/or
R² is H, a methyl or ethyl group and/or
R³, R⁴ each mean, independently of each other, a methylene, ethylene, trimethylene, p-phenylene, ethylidene, 1-methylene-ethane-1,2-diyl group or are absent and/or
R⁵ is a methylene, ethylene, trimethylene, ethene-1,2-diyl, methylethylene, prop-1-ene-1,3-diyl, or a cyclopropylidene group monosubstituted in the 2 position or is absent and/or
R⁶ is H and/or
Z¹, Z² each mean, independently of each other, CO-O, O-CO-NH or O or are absent and/or
Y¹, Y² each mean, independently of each other, O, CO-O or CO-NR⁸ or are absent and/or
R⁷, R⁸ each mean, independently of each other, H, or a methyl or ethyl group and/or
X is H, CN, COOR¹¹ or CONR₂¹² and/or
R⁹, R¹⁰, R¹¹, R¹² each mean, independently of each other, H or a methyl, ethyl or phenyl group and/or
R¹³ is H or a methyl group,
R¹⁴ is H or a vinyl or phenyl group.

6. Composition according to claim 4, **characterized in that**
n is 1,
m is 1,
p is 0,
R is a C₁ to C₃ alkylene or phenylene group,
R² is H,
R⁴ is a branched or straight-chain C₁ to C₆ alkylene group that can be substituted by 1 to 2 fluorine atoms and/or 1 phenyl group, or is absent,
R⁵ is a 1-methylene-ethane-1,2-diyl group,
Z² is absent,
Y² is O or is absent,
X is COOR¹¹ and
R¹¹ is H or a C₁ to C₅ alkyl or phenyl group.

7. Composition according to claim 4, **characterised in that**
n is 2,
m is 2,
p is 1,
R is a quadrivalent aliphatic, aromatic, or aliphatic-aromatic hydrocarbon group with 2 to 12 carbon atoms,
R¹ is absent,
R² is H,
R³ is a C₁ to C₃ alkylene or phenylene group or is absent,
R⁴ is a branched or straight-chained C₁ to C₆ alkylene group which can be substituted by 1 to 2 fluorine atoms and/or 1 phenyl group, or is absent,
R⁵ is a 1-methylene-ethane-1,2-diyl group,
Z¹, Z² are absent,
Y¹ is absent,
Y² is O or is absent,
X is COOR¹¹ and
R¹¹ is H or a C₁ to C₅ alkyl or phenyl group.

8. Composition according to one of claims 3 to 7 comprising maleic acid and/or trichloroacetic acid as the carboxylic acid.

9. Composition according to one of claims 3 to 8 comprising sulfosalicylic acid (2-hydroxy-5-sulfobenzoic acid) as the sulphonic acid.

10. Composition according to one of claims 1 to 9 comprising 1 to 4 different acids.

11. Composition according to one of claims 1 to 10 comprising a polysaccharide, a polyethylene glycol, a polyacrylic acid, a polyacrylamide, a polyvinyl pyrrolidine or a mixture of these substances as the polymer.

12. Composition according to claim 11 comprising a mixture of polyethylene glycol dimethacrylate and polyacrylic acid as the polymer.

13. Composition according to one of claims 1 to 12 further comprising fluoride ions.

14. Composition according to one of claims 1 to 13 further comprising a potassium ion-releasing compound.

15. Composition according to one of claims 1 to 14 further comprising a film-forming component.

16. Composition according to claim 15 comprising hydroxypropyl cellulose.

17. Composition according to one of claims 1 to 16 comprising
| | |
|---|---|
| 0.5 to 40 wt.% | phosphonic acid and/or |
| 1.0 to 40 wt.% | carboxyl and/or hydroxyl-group-containing polymer |
| and/or | |
| 0.5 to 30 wt.% | of a film-forming component and/or |
| 0.1 to 1.0 wt.% | fluoride ions and/or |
| 0.1 to 10 wt.% | potassium ions and |
| 0 to 97.8 wt.% | solvent. |

18. Composition according to claim 17 further comprising 0.1 to 1.0 wt.% flavourings.

19. Composition according to claim 17 or 18 comprising a mixture of ethanol and water as the solvent.

20. Composition according to one of claims 17 to 19 comprising
| | |
|---|---|
| 1 to 5 wt.% | of at least one phosphonic acid, |
| 3 to 7 wt.% | polyacrylic acid, |
| 15 to 25 wt.% | polyethylene glycol dimethacrylate, |
| 3 to 7 wt.% | hydroxypropyl cellulose, |
| 0.1 to 1.0 wt.% | potassium fluoride, |
| 0.05 to 0.2 wt.% | flavouring and |
| 53.8 to 76.9 wt.% | ethanol/water mixture (approx. 50 wt.%). |

21. Kit for manufacturing a dental composition according to one of claims 1 to 20 comprising an acid having protein- and calcium-precipitating properties, and an organic, carboxyl and/or hydroxyl-group-containing polymer in spatially separated form.

22. Kit according to claim 21, **characterized in that** the acid is applied to a brush.

23. Kit according to claim 21 or 22 comprising a solution of the polymer, the composition of which is measured such that, when the solution is combined with the acid of the kit, a composition according to one of claims 18 to 20 is obtained.

24. Kit according to one of claims 21 to 23 wherein the acid and polymer are housed in different chambers of a double-chambered vessel.

25. Use of a composition as defined in claims 1 to 20 for the precipitation of protein.

26. Use of a composition as defined in claims 1 to 20 for the desensitisation of teeth.

27. Use of a composition according to one of claims 1 to 20 for the preparation of an agent for the desensitisation of teeth.

## Revendications

1. Composition dentaire, **caractérisée en ce qu'**elle contient un acide, doté du pouvoir de faire précipiter des protéines et du calcium, et un polymère organique comportant des groupes carboxyle et/ou hydroxyle, et **en ce qu'**elle présente un pH qui vaut de 1 à 3,5.

2. Composition conforme à la revendication 1, **caractérisée en ce qu'**elle contient un acide qui, dans l'eau ou dans un mélange de 50 % en poids d'eau et 50 % en poids d'éthanol, présente une solubilité de 0,5 à 20 % en poids.

3. Composition conforme à l'une des revendications 1 et 2, **caractérisée en ce qu'**elle contient, en tant qu'acide(s), un acide carboxylique, un acide sulfonique et/ou un acide phosphonique.

4. Composition conforme à la revendication 3, **caractérisée en ce qu'**elle contient un acide phosphonique de formule : 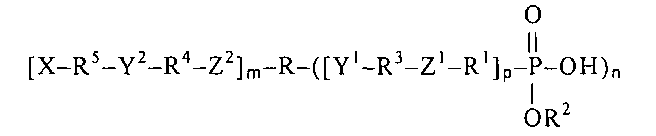 dans laquelle
- l'indice n vaut 1, 2, 3 ou 4 ;
- l'indice m vaut 0, 1 ou 2 ;
- l'indice p vaut 0 ou 1 ;
- R représente un reste d'hydrocarbure aliphatique, linéaire ou ramifié, comportant 1 à 12 atomes de carbone, ou un reste d'hydrocarbure aromatique, comportant 6 à 12 atomes de carbone, ou un reste d'hydrocarbure aliphatique-aromatique, comportant 7 à 16 atomes de carbone, lesquels restes peuvent porter un ou des substituant(s) symbolisé(s) par OH, NH₂ et/ou COOR⁶ ;
- R¹ représente un groupe de type alcane-diyle en C₁₋₁₂, cycloalcane-diyle en C₄₋₁₂, arène-diyle en C₆₋₁₂ ou (alkyl-arène)-diyle en C₇₋₁₆, qui peut porter un ou des substituant(s) symbolisé(s) par OH, NH₂ et/ou COOR⁶, ou ne représente rien ;
- R² représente un atome d'hydrogène, un groupe alkyle en C₁₋₆ ou un groupe phényle ;
- R³ et R⁴, indépendamment l'un de l'autre, représentent chacun un groupe de type alcane-diyle en C₁₋₁₂, arène-diyle en C₆₋₁₂ ou (alkyl-arène--diyle en C₇₋₁₆, qui peut porter un ou des substituant(s) méthyle, phényle ou fluoro, ou ne représentent rien ;
- R⁵ représente un groupe symbolisé par -CH=CR¹³-, un groupe prop-1-ène-1,3-diyle, alcène-diyle en C₁₋₆, cycloalcane-diyle en C₃₋₉, alcane-diyle en C₁₋₆ ou phénylène, ou un groupe de formule 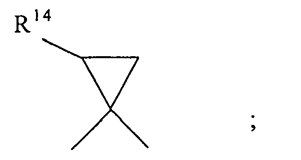
- R⁶ représente un atome d'hydrogène, un groupe alkyle en C₁₋₆ ou un groupe phényle ;
- Z¹ et Z², indépendamment l'un de l'autre, représentent chacun un chaînon ou fragment symbolisé par CO-O, CO-NR⁷, O-CO-NH, O, NH ou S, ou ne représentent rien ;
- Y¹ et Y², indépendamment l'un de l'autre, représentent chacun un chaînon ou fragment symbolisé par O, CO-O, CO-NR⁸ ou O-CO-NH, ou ne représentent rien ;
- R⁷ et R⁸, indépendamment l'un de l'autre, représentent chacun un atome d'hydrogène ou un groupe alkyle en C₁₋₆ ;
- X représente un atome d'hydrogène ou un groupe symbolisé par CN, N(R⁹)₂, OR¹⁰, COOR¹¹ ou CON(R¹²)₂ ;
- R⁹, R¹⁰, R¹¹ et R¹², indépendamment les uns des autres, représentent chacun un atome d'hydrogène, un groupe alkyle en C₁₋₁₀ ou un groupe phényle ;
- R¹³ représente un atome d'hydrogène ou un groupe méthyle ;
- et R¹⁴ représente un atome d'hydrogène, un groupe alkyle en C₁₋₁₀, un groupe vinyle ou un groupe phényle.

5. Composition conforme à la revendication 4, **caractérisée en ce que** :
- l'indice n vaut 1 ou 2 ;
- et/ou l'indice m vaut 1 ;
- et/ou l'indice p vaut 0 ;
- et/ou R représente un reste d'alcane, aliphatique, linéaire ou ramifié, monovalent à pentavalent, comportant 1 à 7 atomes de carbone, ou un reste d'hydrocarbure aromatique, comportant 6 atomes de carbone, ou un reste d'hydrocarbure aliphatique-aromatique, comportant 8 atomes de carbone ;
- et/ou R¹ représente un groupe méthylène ou éthylène ou ne représente rien ;
- et/ou R² représente un atome d'hydrogène ou un groupe méthyle ou éthyle ;
- et/ou R³ et R⁴, indépendamment l'un de l'autre, représentent chacun un groupe méthylène, éthylène, triméthylène, para-phénylène, éthylidène ou 1-méthylène-éthane-1,2-diyle, ou ne représentent rien ;
- et/ou R⁵ représente un groupe méthylène, éthylène, triméthylène, éthène-1,2-diyle, méthyl-éthylène ou prop-1-ène-1,3-diyle, ou un groupe cyclopropylidène portant un unique substituant en position 2, ou ne représente rien ;
- et/ou R⁶ représente un atome d'hydrogène ;
- et/ou Z¹ et Z², indépendamment l'un de l'autre, représentent chacun un chaînon ou fragment symbolisé par CO-O, O-CO-NH ou O, ou ne représentent rien ;
- et/ou Y¹ et Y², indépendamment l'un de l'autre, représentent chacun un chaînon ou fragment symbolisé par O, CO-O ou CO-NR⁸, ou ne représentent rien ;
- et/ou R⁷ et R⁸, indépendamment l'un de l'autre, représentent chacun un atome d'hydrogène ou un groupe méthyle ou éthyle ;
- et/ou X représente un atome d'hydrogène ou un groupe symbolisé par CN, COOR¹¹ ou CON(R¹²)₂ ;
- et/ou R⁹, R¹⁰, R¹¹ et R¹², indépendamment les uns des autres, représentent chacun un atome d'hydrogène ou un groupe méthyle, éthyle ou phényle ;
- et/ou R¹³ représente un atome d'hydrogène ou un groupe méthyle ;
- et/ou R¹⁴ représente un atome d'hydrogène ou un groupe vinyle
ou phényle.

6. Composition conforme à la revendication 4, **caractérisée en ce que** :
- l'indice n vaut 1 ;
- l'indice m vaut 1 ;
- l'indice p vaut 0 ;
- R représente un groupe alcane-diyle en C₁₋₃ ou phénylène ;
- R² représente un atome d'hydrogène ;
- R⁴ représente un groupe alcane-diyle en C₁₋₆, linéaire ou ramifié, qui peut porter en tant que substituant(s) un ou deux atomes de fluor et/ou un groupe phényle, ou ne représente rien ;
- R⁵ représente un groupe 1-méthylène-éthane-1,2-diyle ;
- Z² ne représente rien ;
- Y² représente un chaînon symbolisé par O, ou ne représente rien ;
- X représente un groupe symbolisé par COOR¹¹ ;
- et R¹¹ représente un atome d'hydrogène ou un groupe alkyle en C₁₋₅ ou phényle.

7. Composition conforme à la revendication 4, **caractérisée en ce que** :
- l'indice n vaut 2 ;
- l'indice m vaut 2 ;
- l'indice p vaut 1 ;
- R représente un reste tétravalent d'hydrocarbure aliphatique, aromatique ou aliphatique-aromatique, comportant 2 à 12 atomes de carbone ;
- R¹ ne représente rien ;
- R² représente un atome d'hydrogène ;
- R³ représente un groupe alcane-diyle en C₁₋₃ ou phénylène, ou ne représente rien ;
- R⁴ représente un groupe alcane-diyle en C₁₋₆, linéaire ou ramifié, qui peut porter en tant que substituant(s) un ou deux atomes de fluor et/ou un groupe phényle, ou ne représente rien ;
- R⁵ représente un groupe 1-méthylène-éthane-1,2-diyle ;
- Z¹ et Z² ne représentent rien ;
- Y¹ ne représente rien ;
- Y² représente un chaînon symbolisé par O, ou ne représente rien ;
- X représente un groupe symbolisé par COOR¹¹ ;
- et R¹¹ représente un atome d'hydrogène ou un groupe alkyle en C₁₋₅ ou phényle.

8. Composition conforme à l'une des revendications 3 à 7, **caractérisée en ce qu'**elle contient, en tant qu'acide carboxylique, de l'acide maléique et/ou de l'acide trichloracétique.

9. Composition conforme à l'une des revendications 3 à 8, **caractérisée en ce qu'**elle contient, en tant qu'acide sulfonique, de l'acide sulfo-salicylique (acide 2-hydroxy-5-sulfo-benzoïque).

10. Composition conforme à l'une des revendications 1 à 9, **caractérisée en ce qu'**elle contient un à quatre acides différents.

11. Composition conforme à l'une des revendications 1 à 10, **caractérisée en ce qu'**elle contient, en tant que polymère, un polysaccharide, un polyéthylèneglycol, un poly(acide acrylique), un polyacrylamide, une poly(vinyl-pyrrolidone) ou un mélange de tels polymères.

12. Composition conforme à la revendication 11, **caractérisée en ce qu'**elle contient, en tant que polymère, un mélange de diméthacrylate de polyéthylèneglycol et de poly(acide acrylique).

13. Composition conforme à l'une des revendications 1 à 12, **caractérisée en ce qu'**elle contient en outre des ions fluorure.

14. Composition conforme à l'une des revendications 1 à 13, **caractérisée en ce qu'**elle contient en outre un composé qui libère des ions de potassium.

15. Composition conforme à l'une des revendications 1 à 14, **caractérisée en ce qu'**elle contient en outre un composant filmogène.

16. Composition conforme à la revendication 15, **caractérisée en ce qu'**elle contient une hydroxypropyl-cellulose.

17. Composition conforme à l'une des revendications 1 à 16, **caractérisée en ce qu'**elle contient :
- 0,5 à 40 % en poids d'acide phosphorique,
- et/ou 1,0 à 40 % en poids de polymère comportant des groupes carboxyle et/ou hydroxyle,
- et/ou 0,5 à 30 % en poids d'un composant filmogène,
- et/ou 0,1 à 1,0 % en poids d'ions fluorure,
- et/ou 0,1 à 10 % en poids d'ions de potassium,
- et/ou 0 à 97,8 % en poids de solvant.

18. Composition conforme à la revendication 17, **caractérisée en ce qu'**elle contient en outre 0,1 à 1,0 % en poids d'un agent de sapidité.

19. Composition conforme à la revendication 17 ou 18, **caractérisée en ce qu'**elle contient, en tant que solvant, un mélange d'éthanol et d'eau.

20. Composition conforme à l'une des revendications 17 à 19, **caractérisée en ce qu'**elle contient :
- 1 à 5 % en poids d'au moins un acide phosphonique,
- 3 à 7 % en poids de poly(acide acrylique),
- 15 à 25 % en poids de diméthacrylate de polyéthylèneglycol,
- 3 à 7 % en poids d'hydroxypropyl-cellulose,
- 0,1 à 1,0 % en poids de fluorure de potassium,
- 0,05 à 0,2 % en poids d'un agent de sapidité,
- et 53,8 à 76,9 % en poids d'un mélange éthanol/eau à environ 50 % en poids.

21. Trousse pour la préparation d'une composition conforme à l'une des revendications 1 à 20, contenant un acide, doté du pouvoir de faire précipiter des protéines et du calcium, et, physiquement séparé de cet acide, un polymère organique comportant des groupes carboxyle et/ou hydroxyle.

22. Trousse conforme à la revendication 21, **caractérisée en ce que** l'acide est déposé sur un pinceau.

23. Trousse conforme à la revendication 21 ou 22, **caractérisée en ce qu'**elle contient une solution du polymère dont la composition est conçue de manière à ce que, lorsqu'on réunit cette solution et l'acide de la trousse, on obtienne une composition conforme à l'une des revendications 18 à 20.

24. Trousse conforme à l'une des revendications 21 à 23, **caractérisée en ce que** l'acide et le polymère sont placés respectivement dans l'un et l'autre compartiments d'un contenant à deux compartiments.

25. Utilisation d'une composition conforme à l'une des revendications 1 à 20 pour faire précipiter des protéines.

26. Utilisation d'une composition conforme à l'une des revendications 1 à 20 pour la désensibilisation des dents.

27. Utilisation d'une composition conforme à l'une des revendications 1 à 20 pour la préparation d'un agent de désensibilisation des dents.
